(19) Europäisches Patentamt
European Patent Office
Office européen des brevets

(11) EP 3 820 879 B1

(12) EUROPEAN PATENT SPECIFICATION

(45) Date of publication and mention of the grant of the patent:
**06.11.2024 Bulletin 2024/45**

(21) Application number: **19827511.7**

(22) Date of filing: **26.06.2019**

(51) International Patent Classification (IPC):
***C07H 19/06*** (2006.01) ***C07H 1/00*** (2006.01)
***A61K 31/7068*** (2006.01) ***A61P 31/18*** (2006.01)
***A61P 31/14*** (2006.01) ***A61P 31/20*** (2006.01)
***A61P 31/22*** (2006.01) ***A61P 31/12*** (2006.01)

(52) Cooperative Patent Classification (CPC):
**A61K 31/7068; A61P 31/12; A61P 31/14; A61P 31/18; A61P 31/20; A61P 31/22; C07H 1/02; C07H 19/06; C07H 19/073; C07H 19/10; C07H 19/11**

(86) International application number:
**PCT/CN2019/093001**

(87) International publication number:
**WO 2020/001475 (02.01.2020 Gazette 2020/01)**

# (54) PHOSPHORUS-CONTAINING PRODRUGS OF GEMCITABINE

PHOSPHORHALTIGE PRODRUGS VON GEMCITABIN

PROMÉDICAMENTS DE GEMCITABINE CONTENANT DU PHOSPHORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2018 PCT/CN2018/093564**

(43) Date of publication of application:
**19.05.2021 Bulletin 2021/20**

(73) Proprietor: **Changchun Changcheng Pharmaceutical Technology Co., LTD**
**Changchun City, Jilin Province (CN)**

(72) Inventor: **XU, Mingyan**
**Changchun Jilin 130000 (CN)**

(74) Representative: **Hoffmann Eitle Patent- und Rechtsanwälte PartmbB**
**Arabellastraße 30**
**81925 München (DE)**

(56) References cited:
**CN-A- 102 351 931**
**CN-A- 104 086 612**
**CN-A- 104 211 742**
**US-A1- 2013 131 008**

- **DAVID M. BENDER ET AL: "Synthesis, Crystallization, and Biological Evaluation of an Orally Active Prodrug of Gemcitabine", JOURNAL OF MEDICINAL CHEMISTRY, vol. 52, no. 22, 26 November 2009 (2009-11-26), pages 6958 - 6961, XP055058643, ISSN: 0022-2623, DOI: 10.1021/jm901181h**
- **BOYER, SERGE H. ET AL.: "Synthesis and Characterization of a Novel Liver-Targeted Prodrug of Cytosine-1-Beta-D-arabinofuranoside Monophosphate for the Treatment of Hepatocellular Carcinoma", JOURNAL OF MEDICINAL CHEMISTRY, vol. 49, no. 26, 21 December 2006 (2006-12-21), pages 7711 - 7720, XP055669950**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

This invention relates to phosphorus-containing prodrugs of gemcitabine and their use in the treatment of cancer.

### BACKGROUND OF THE INVENTION

Gemcitabine hydrochloride (2',2'-difluoro-2'-deoxycytidine hydrochloride) is an anti-tumor agent marketed as Gemzar® for the treatment of various cancers, such as pancreatic cancer, breast cancer and non-small cell lung dancer (NSCLC) and is being evaluated for ovarian cancer. Moreover, gemcitabine may also be used in the treatment of HCV as well as a modulator of immune function (see US Patent No 6,555,518). In another aspect, combination of gemcitabine with decitabine was shown to potently inhibit HIV-1 through a mechanism that is distinct from the mechanisms for the drugs currently used to treat HIV-1 infection (Antimicrobial Agents and Chemotherapy, 2012, 56, 1942-1948). Gemcitabine also demonstrated antiviral activities against other viruses such as polioviruses (ACS Infect. Dis., 2017, 3(1), 45-53), human rhinovirus (Antiviral Research, 2017, 145, 6-13), and influenza A viruses (J. Biol. Chem., 2012, 287(42), 35324-35332).

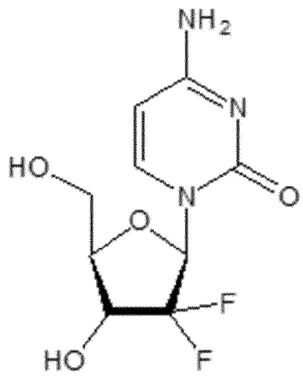

Gemcitabine

Gemzar® is currently administered by intravenous infusion at a dose of approximately 1000 to 1250 mg/m over 30 minutes, once weekly for up to 7 weeks followed by a week of rest from the treatment.

The oral administration of gemcitabine may be limited by its poor oral bioavailability due to first pass metabolism. Shipley LA. et. al., "Metabolism and disposition of gemcitabine, and oncolytic deoxycytidine analog, in mice, mice, and dogs". Drug Metabolism & Disposition, 1992, 20(6):849-55. In addition, when dosed orally, gemcitabine is implicated in causing adverse dose-limiting intestinal lesions characterized by moderate-to-marked loss of mucosal epithelium (atrophic enteropathy) throughout the entire length of the intestinal tract in mice which have been given a single oral (gavage) gemcitabine dose of 167, 333, or 500 mg/kg. Horton ND et. al., "Toxicity of single-dose oral gemcitabine in mice", American Association for Cancer Research, Poster Presentation, Orlando, FL, March 27-31, 2004. Comparable exposures via intravenous dosing in previous mouse studies did not result in death or gastrointestinal toxicity.

Various prodrugs and sustained released formulations of gemcitabine have been explored to find improvements. Examples of such prodrugs and sustained released formulations can be found in WO 04/0412303 "Gemcitabine Prodrugs, Pharmaceutical Compositions and Uses Thereof', Gallop et. al.; WO 98/32762 "Gemcitabine Derivatives," Myhren, Finn, et al.; WO 02/09768 "Therapeutic polyesters and polyamides," Uhrich, Kathryn E.; WO 02/76476 "Prodrugs of anticancer agents based on substituted aromatic acids," Greenwald, Richard m, et al.; WO 02/65988, "Terminallybranched polymeric lilüers and polymeric conjugates as prodrug," Choe, Yun Hwang, *et al.*

Gemcitabine amide derivatives have been described in the art as useful intermediates in the synthesis of gemcitabine (see e.g. Britton, et al., U.S. Pat. No. 5,420,266 and Grindey, et al., U.S. Pat. No. 5,464,826) and also useful as prodrug moieties for the administration of gemcitabine. See e.g. Gallop, et al., WO 04/041203.

LY2334737, an amide prodrug of Gemcitabine, was reported as an oral dosing agent (J. Med. Chem., 2009, 52, 6958-6961). More importantly, it has shown clinical benefits in phase I human clinical trials (Invest. New Drugs, 2015, 33, 1206-1216 and references cited therein). Hepatotoxicity, however, was observed in some patients and was suggested to possibly be associated with genetic polymorphism of the cytidine deaminase gene (rs818202).

Hepatocellular Carcinoma (HCC) is a cancer difficult to treat. The combinations of gemcitabine with other anticancer agents (e.g. doxorubicin and oxaliplatin) have shown promising results in improving overall survival (Am. J. Clin.

Oncol., 2012, 35(5), 418-423 and references cited therein) which remains as the most important endpoint for HCC. In another report, the combination of gemcitabine and docetaxel in late stage liver cancer patients showed significant anti-cancer activity (Cancer Research on Prevention and Treatment, 2012, 39(11), 1369-1372). Moreover, gemcitabine chemotherapy in middle to late stage liver cancer patients also showed improvement of immune functions (Journal of Hainan Medical University, 2016, 22(17), 2029-2031), which should make gemcitabine a potential partner for combination therapy with immuno-oncology products such as check point inhibitors PD-1, PD-L1, CTLA4 antibodies.

Liver-targeted prodrugs of gemcitabine will deliver gemcitabine active metabolites to the liver selectively, and thus could be used to treat liver cancers. As such, there is need to develop liver-targeted prodrugs of gemcitabine that could allow oral dosing, pass through the intestinal tract intact without substantial degradation and deliver gemcitabine to the afflicted area in liver with acceptable safety and efficacy.

The oral delivery of liver-targeted prodrugs of gemcitabine could also be used to treat other solid tumors (such as cancers of the lung, pancreas, colon, prostate, breast, etc.) either as a monotherapy or as part of a combination therapy. For example, the combination of gemcitabine with albumin-bound taxol as a first line therapy for late stage pancreatic cancer patients showed clear therapeutic benefits with DCR of 77.8%; PFS and OS of 5 and 8 months, respectively (Chinese Clinical Oncology, 2016, 21(7), 642-645).

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

## BRIEF SUMMARY OF THE INVENTION

After extensive work, we discovered various novel phosphoramide derivatives of gemcitabine as prodrugs. These prodrugs pass through the gastrointestinal (GI) tract substantially intact and are converted into gemcitabine inside liver, thus minimizing the formation of deoxydifluorouridine (dFdU), the predominant gemcitabine metabolite, in the GI, liver, and plasma. The significantly lower levels of dFdU (in both liver and plasma) observed with these phosphorus-containing prodrugs compared to gemcitabine itself (either i.v. or p.o. dosing) should translate into much less toxicity compared to gemcitabine while maintaining appropriate efficacy and safety profiles when administered orally or intravenously.

Thus, the present invention aims to provide novel phosphorus-containing prodrugs of gemcitabine, which are capable of being given either orally or intravenously and delivering gemcitabine active metabolites to the liver selectively while minimizing the formation of dFdU, as further defined in the claims. When the prodrugs are given orally they traverse the intestinal tract substantially intact into the portal bloodstream with less gastrointestinal toxicity and better bioavailability than with the parent drug (i.e., gemcitabine, aka dFdC) and maintaining the efficacy of the parent drug at lower doses. Therefore, these prodrugs can not only deliver gemcitabine to the liver but also reach other organs since the blood leaving the liver will carry gemcitabine into other organs. It is contemplated that in addition to treating liver cancer these phosphorus-containing gemcitabine prodrugs could be also useful to treat cancers of other organs such as pancreas, lung, prostate etc, namely susceptible neoplasms, wherein the susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC).

According to a first aspect of the present invention, there is provided a compound of formula I:

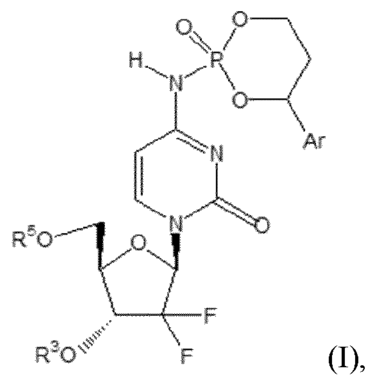

(I),

its stereoisomer, salt, hydrate, solvate, or crystalline form thereof;

wherein,

Ar is optionally substituted phenyl, or optionally substituted 3-pyridyl or 4-pyridyl, $R^3$ is hydrogen or isobutyryl, and $R^5$ is hydrogen or isobutyryl, or $R^3$ and $R^5$ together form a cyclic group as shown below:

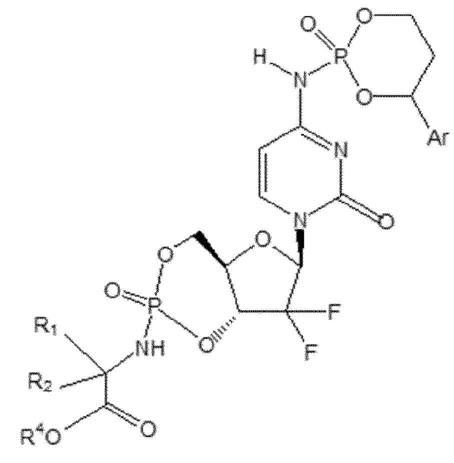

wherein: $R^1$ and $R^2$ are independently selected from the group consisting of H, alkyl and alkylaryl, or $R^1$ and $R^2$ together form an alkylene chain so as to provide, together with the C atom to which they are attached, a cyclic system; and

$R^4$ is selected from the group consisting of alkyl, aryl and alkylaryl.

In another preferred embodiment of the first aspect of the invention, $R^1$ and $R^2$ are independently selected from the group consisting of H, methyl, benzyl and $-CH_2CH(CH_3)_2$, or $R^1$ and $R^2$ together with the C atom to which they are attached, provide a $C_{5-6}$ ring, preferably a pentyl ring.

In another preferred embodiment of the first aspect of the invention, Ar is 3-chlorophenyl, 3-pyridyl, or 4-pyridyl.

In another preferred embodiment of the first aspect of the invention, the compound according to formula I is selected from the group consisting of:

1-((2R,4R,5R)-3,3-difluoro-4-hydroxy-5-(hydroxymethyl)tetrahydrofuran-2-yl)-4-((2-oxido-4-(pyridin-4-yl)-1,3,2-dioxaphosphinan-2-yl)amino)pyrimidin-2(1H)-one;

1-((2R,4R,5R)-3,3-difluoro-4-hydroxy-5-(hydroxymethyl)tetrahydrofuran-2-yl)-4-((2-oxido-4-(pyridin-3-yl)-1,3,2-dioxaphosphinan-2-yl)amino)pyrimidin-2(1H)-one;

4-((4-(3-chlorophenyl)-2-oxido-1,3,2-dioxaphosphinan-2-yl)amino)-1-((2R,4R,5R)-3,3 -difluoro-4-hydroxy-5-(hydroxymethyl)tetrahydrofuran-2-yl)pyrimidin-2(1H)-one;

4-((4-(3-bromophenyl)-2-oxido-1,3,2-dioxaphosphinan-2-yl)amino)-1-((2R,4R,5R)-3,3-difluoro-4-hydroxy-5-(hydroxymethyl)tetrahydrofuran-2-yl)pyrimidin-2(1H)-one;

1-((2R,4R,5R)-3,3-difluoro-4-hydroxy-5-(hydroxymethyl)tetrahydrofuran-2-yl)-4-((4-(3-fluorophenyl)-2-oxido-1,3,2-dioxaphosphinan-2-yl)amino)pyrimidin-2(1H)-one;

1-((2R,4R,5R)-3,3-difluoro-4-hydroxy-5-(hydroxymethyl)tetrahydrofuran-2-yl)-4-((4-(4-methoxyphenyl)-2-oxido-1,3,2-dioxaphosphinan-2-yl)amino)pyrimidin-2(1H)-one; and

((2R,3R,5R)-5-(4-((4-(3-chloro-4-methoxyphenyl)-2-oxido-1,3,2-dioxaphosphinan-2-yl)amino)-2-oxopyrimidin-1(2H)-yl)-4,4-difluoro-3-hydroxytetrahydrofuran-2-yl)methyl pivalate (2R,3R,5R)-5-(4-((4-(3-chlorophenyl)-2-oxido-l,3,2-dioxaphosphinan-2-yl)amino)-2-oxopyrimidin-1(2H)-yl)-4,4-difluoro-2-((isobutyryloxy)methyl)tetrahydrofuran-3-yl isobutyrate ((2R,3R,5R)-3-acetoxy-5-(4-((4-(3-chlorophenyl)-2-oxido-1,3,2-dioxaphosphinan-2-yl)amino)-2-oxopyrimidin-1(2H)-yl)-4,4-difluorotetrahydrofuran-2-yl)methyl acetate;

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((propionyloxy)methyl)tetrahydrofuran-3-yl propionate;

(2F,3F,5R)-5-(4-((4-(3-chlorophenyl)-2-oxido-1,3,2-dioxaphosphinan-2-yl)amino)-2-oxopyrimidin-1(2H)-yl)-4,4-difluoro-2-((2-methoxyacetoxy)methyl)tetrahydrofuran-3-yl 2-methoxyacetate;

(2R,3R, SR)-5-(4-((4-(3-chlorophenyl)-2-oxido-1,3,2-dioxaphosphinan-2-yl)amino)-2-oxopyrimidin-1 (2H)-yl)-4,4-difluoro-2-((pivaloyloxy)methyl)tetrahydrofuran-3-yl pivalate; and (2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((isopropyloxycarbonyloxy)methyl)tetrahydrofuran-3-yl isopropyl carbonate.

Another aspect of the invention provides a method of treating susceptible neoplasms in a mammal comprising

administering to a mammal in need thereof a therapeutically effective amount of a compound according to formula I. The susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC).

The present invention also provides use of a compound according to formula I for the manufacture of a medicament for the treatment of susceptible neoplasms, wherein the susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC).

The present invention further provides a compound according to formula I for use in the treatment of susceptible neoplasms, wherein the susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC).

The present invention further provides a pharmaceutical composition comprising a compound according to formula I, as defined in the claims in combination with a pharmaceutically acceptable carrier, diluent or excipient.

Another aspect of the invention provides a method of preparing a pharmaceutical composition comprising the step of combining a compound according to formula I as defined in the claims with a pharmaceutically acceptable carrier, diluent or excipient.

In addition, the present invention also provides a method for preparing compounds of formula I as defined in the claims, comprising the following steps:

(i) protecting 5'-position (5'-OH) of gemcitabine with a suitable protection group to obtain a compound of formula (IV), wherein PG represents a suitable protection group for -OH group;

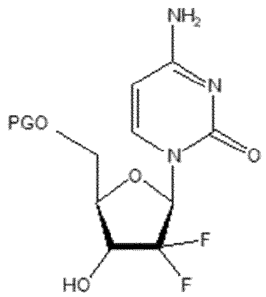

(IV)

(ii) modifying the compound of formula (IV) (for example, by reacting the compound of formula (IV) with a compound of formula (V)) to obtain a compound of formula (III) (i.e., preparing N-prodrugs of gemcitabine), wherein Ar is as defined herein;

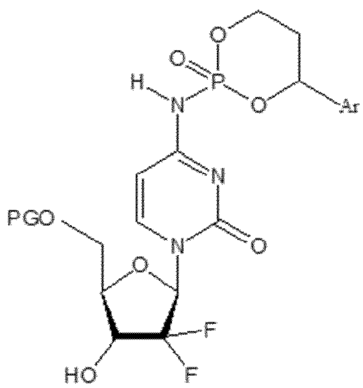

(III)

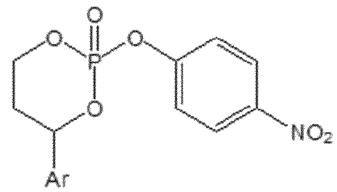

(V)

(iii) removing the protection group (PG) at 5'-position (5'-OH) of gemcitabine to obtain a compound of formula (II);

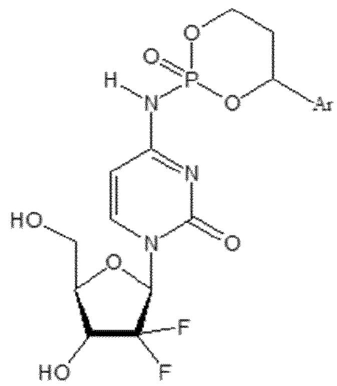

(II)

and, optionally further

(iv) modifying the compound of formula (II) at 3'-position (3'-OH) and/or 5'-position (5'-OH) to obtain the compound of formula (I), wherein Ar, $R^3$ and $R^5$ are as defined in the claims.

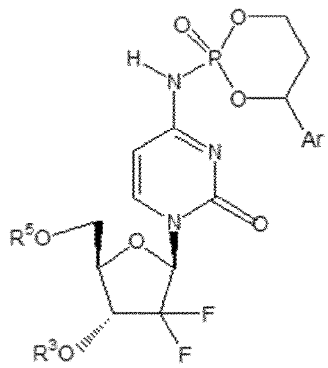

(I).

Furthermore, the present invention provides a method of treating susceptible neoplasms in a mammal as defined in the claims comprising administering to a mammal in need thereof a therapeutically effective amount of a compound according to formula I in combination with at least one (preferably one or two) oncolytic agent and/or immune-oncology agent.

Preferably, the oncolytic agent is selected from the group consisting of 5-fluorouracil, chloroquine, S-1 (the combination drug tegafur/gimeracil/oteracil, Liu TW, et al. Lancet Oncol. 2016, 17: 12-4), vinorelbine, sorafenib, elpamotide, capecitabine, carboplatin, cisplatin, oxaliplatin, aurora kinase inhibitors (e.g. MSC1992371A, Investigational New Drugs, 2014, 32(1), 94-103), EGFR inhibitors (e.g. erlotinib, gefitinib), tyrosine kinase inhibitors (e.g. lapatinib, vandetanib), topoisomerase inhibitors (e.g. irinotecan, exatecan, Indotecan (LMP400) and Indimitecan (LMP776), nab-paclitaxel, paclitaxel, docetaxel, pemetrexed, curcumin and radiation therapy.

Preferably, the immune-oncology agent is selected from the group consisting of checkpoint inhibitors, PD-1, PD-L1, CTLA-4 and VEGF-A antibodies.

## BRIEF DESCRIPTION OF THE DRAWINGS

Figure 1. Plasma exposures of **Compound 4** and gemcitabine (aka dFdC) following i.v. administration (equivalent dose to 15 mg/kg of gemcitabine) or PO administration (80 mg/kg) of Compound **4**; and

Figure 2. Liver concentrations of dFdC and dFdU at 2h following administration of Compound 4 (iv, equivalent dose to 15 mg/kg of gemcitabine; PO, 80 mg/kg) and Gemcitabine (iv, 15 mg/kg).

## DETAILED DESCRIPTION OF THE INENTION

### Definitions

The terms as used herein are to be construed with ordinary and typical meaning to those of ordinary skill in the art. However, the following terms are given the particular definition as defined below.

The term "alkyl" means a branched or unbranched, cyclic or acyclic, saturated or unsaturated (e.g., alkenyl or alkynyl) hydrocarbyl radical. Where cyclic, the alkylene group is preferably $C_3$ to $C_{12}$, more preferably $C_5$ to $C_{10}$, more preferably $C_5$ to $C_7$. Where acyclic, the alkyl group is preferably $C_1$ to $C_{16}$, more preferably $C_1$ to $C_6$ saturated alkyl, such as methyl, ethyl, propyl, butyl, pentyl and hexyl.

The term "aryl" refers to an aromatic group containing 5 to 14 ring atoms, for example phenyl or naphthyl.

The term "heteroaryl" refers to an aryl group containing one, two, three or four, preferably one, heteroatoms independently selected from the group consisting of O, N and S. Examples of such heteroaryl include pyridyl, pyrrolyl, furanyl and thiophenyl.

The aryl and heteroaryl groups may be substituted or unsubstituted. Where substituted, there will generally be one to three substituents present, preferably one substituent. Substituents may include halogen atoms, by which is meant F, Cl, Br and I atoms, and halomethyl groups such as $CF_3$ and $CCl_3$; oxygen containing groups such as oxo, hydroxy, carboxy, carboxy$C_{1-6}$alkyl, alkoxy, alkoyl, alkoyloxy, aryloxy, aryloyl and aryloyloxy; nitrogen containing groups such as amino, $C_{1-6}$alkylamino, cyano, azide and nitro; sulphur containing groups such as thiol, $C_{1-6}$alkylthiol, sulphonyl and sulphoxide; alkyl groups as defined above, which may themselves be substituted; and aryl groups as defined above, which may themselves be substituted, such as phenyl and substituted phenyl. Substituents on said alkyl and aryl groups are as defined immediately above.

The term "acyl" refers to a radical RCO- derived usually from an organic acid by removal of the hydroxyl from all acid groups, wherein R represents an alkyl group. Preferred examples of acyl include $C_{1-6}$acyl, such as formyl, acetyl, propionyl, butyryl (e.g., isobutyryl).

The terms "alkoxy" and "aryloxy" mean alkyl-O- (for example where alkyl is $C_1$ to $C_{16}$, preferably $C_1$ to $C_6$) and aryl-O- (for example where aryl is a 5 to 14 membered aromatic mono- or bifused ring moiety, optionally containing 1, 2, 3 or 4 heteroatoms selected, independently, from O, S and N, preferably aryl is phenyl), respectively.

The term "alkoxycarbonyl" means alkoxy-C(O)-, preferably $C_{1-16}$alkoxycarbonyl, and more preferably $C_{1-6}$alkoxycarbonyl, for example methylcarbonyl, ethylcarbonyl, propylcarbonyl, and butylcarbonyl.

The terms "alkoyl" and "aryloyl" mean alkyl-CO- (for example where alkyl is $C_1$ to $C_{16}$, preferably $C_1$ to $C_6$) and aryl-CO- (for example where aryl is a 5 to 14 membered aromatic mono or bifused ring moiety, optionally containing 1, 2, 3 or 4 heteroatoms selected, independently, from O, S and N, preferably aryl is phenyl), respectively.

The terms "alkoyloxy" and "aryloyloxy" mean alkyl-CO-O (for example where alkyl is $C_1$ to $C_{16}$, preferably $C_1$ to $C_6$) and aryl-CO-O (for example where aryl is a 5 to 14 membered mono- or bifused aromatic ring system, optionally containing 1, 2, 3 or 4 heteroatoms selected, independently, from O, S and N, preferably aryl is phenyl), respectively.

The term "heterocyclic" refers to a cyclic group containing 1, 2, 3 or 4 heteroatoms selected, independently, from O, S and N, and may be selected from the group consisting of pyrrolyl, imidazolyl, pyraziolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronly, pyridyl, pyrazinyl, pyridazinyl, piperidyl, piperazinyl, orpholinyl, thionaphthyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl.

**Compound of formula I**

An aspect of the invention is directed to a compound represented by formula I, its stereoisomer, pharmaceutically acceptable salt, hydrate, solvate, or crystalline form thereof,

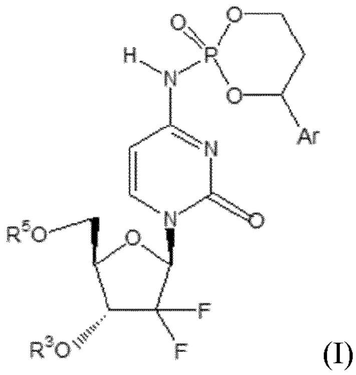

(I)

wherein,

Ar is optionally substituted phenyl, or optionally substituted 3-pyridyl or 4-pyridyl, $R^3$ is hydrogen or isobutyryl, and $R^5$ is hydrogen or isobutyryl,or $R^3$ and $R^5$ together form a cyclic phosphate group as shown below:

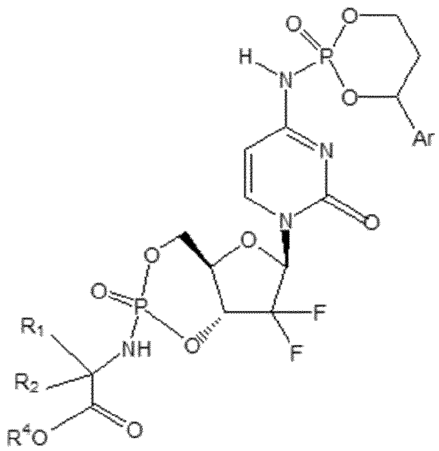

wherein

$R^1$ and $R^2$ are independently selected from the group consisting of H, alkyl and alkylaryl, or $R^1$ and $R^2$ together form an alkylene chain so as to provide, together with the C atom to which they are attached, a cyclic system;

$R^4$ is selected from the group consisting of alkyl, aryl and alkylaryl; and

Preferably, the group Ar is a substituted or unsubstituted phenyl group. Particularly preferred substituents are electron withdrawing groups such as halogen (preferably chlorine or fluorine), trihalomethyl (preferably trifluoromethyl), cyano and nitro groups. For example, Ar can be phenyl, 3-chloro-phenyl, 3,5-dichloro-phenyl, p-trifluoromethyl-phenyl, p-cyano-phenyl, or p-nitro-phenyl. When Ar is a heteroaryl group, preferably it is optionally substituted pyridyl, such as 3-pyridyl, or 4-pyridyl.

$R^3$ is hydrogenor more preferably hydrogen or isobutyryl.

Most preferably, the compound of formula I is selected from the group consisting of:

1-((2R,4R,5R)-3,3-difluoro-4-hydroxy-5-(hydroxymethyl)tetrahydrofuran-2-yl)-4-((2-oxido-4-(pyridin-4-yl)-1,3,2-dioxaphosphinan-2-yl)amino)pyrimidin-2(1H)-one;

1-((2R,4R,5R)-3,3-difluoro-4-hydroxy-5-(hydroxymethyl)tetrahydrofuran-2-yl)-4-((2-oxido-4-(pyridin-3-yl)-1,3,2-dioxaphosphinan-2-yl)amino)pyrimidin-2(1H)-one;

4-(((2R,4S)-4-(3-chlorophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;

4-(((2S,4S)-4-(3-chlorophenyl)-2-oxido-1,3,2-dioxaphosphinan-2-yl)amino)-1-((2R,4R,5R)-3,3-difluoro-4-hydroxy-5-(hydroxymethyl)tetrahydrofuran-2-yl)pyrimidin-2(1H)-one;

4-((4-(3-bromophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;

1-((2R,4R,5R)-3,3-difluoro-4-hydroxy-5-(hydroxymethyl)tetrahydrofuran-2-yl)-4-((4-(3-fluorophenyl)-2-oxido-1,3,2-dioxaphosphinan-2-yl)amino)pyrimidin-2(1H)-one;

1-((2R,4R,5R)-3,3-difluoro-4-hydroxy-5-(hydroxymethyl)tetrahydrofuran-2-yl)-4-((4-(4-methoxyphenyl)-2-oxido-1,3,2-dioxaphosphinan-2-yl)amino)pyrimidin-2(1H)-one; and

((2F,3R,5R)-5-(4-((4-(3-chloro-4-methoxyphenyl)-2-oxido-1,3,2-dioxaphosphinan-2-yl)amino)-2-oxopyrimidin-1(2H)-yl)-4,4-difluoro-3-hydroxytetrahydrofuran-2-yl)methyl pivalate

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((isobutyryloxy)methyl)tetrahydrofuran-3-yl isobutyrate

((2R,3R,5R)-3-acetoxy-5-(4-((4-(3-chlorophenyl)-2-oxido-1,3,2-dioxaphosphinan-2-yl)amino)-2-oxopyrimidin-1(2H)-yl)-4,4-difluorotetrahydrofuran-2-yl)methyl acetate;

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((propionyloxy)methyl)tetrahydrofuran-3-yl propionate;

(2R,3R,5R)-5-(4-((4-(3-chlorophenyl)-2-oxido-1,3,2-dioxaphosphinan-2-yl)amino)-2-oxopyrimidin-1(2H)-yl)-4,4-difluoro-2-((2-methoxyacetoxy)methyl)tetrahydrofuran-3-yl 2-methoxyacetate;

(2R,3R, SR)-5-(4-((4-(3-chlorophenyl)-2-oxido-1,3,2-dioxaphosphinan-2-yl)amino)-2-oxopyrimidin-1 (2H)-yl)-4,4-difluoro-2-((pivaloyloxy)methyl)tetrahydrofuran-3-yl pivalate; and

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((isopropyloxycarbonyloxy)methyl)tetrahydrofuran-3-yl isopropyl carbonate.

**Synthesis of the Compounds of the Invention**

The compounds in this invention may be prepared by the processes described herein, as well as relevant published literature procedures that are used by those skilled in the art. All starting materials and reagents are well known in the art and/or readily commercially available or prepared by methods described herein. A process for preparing gemcitabine (2',2'-difluoro-2'-deoxycytidine), for example, is disclosed in US Patent No. 6,555,518. It should be understood that the following processes are provided solely for the purpose of illustration and do not limit the invention which is defined by the claims. Typically, the synthesis of a compound of formula I includes the following general steps:

(1) Protection of the 5'-position of gemcitabine which could be carried out according to the procedures generally known in the art (e.g., "Protecting Groups in Organic Synthesis," 3rd Edition, Wiley, 1999). For example, selective protection of the 5'-OH could be achieved using TBDPSCI under suitable conditions;

(2) Preparation of a prodrug of the 4-amino group of gemcitabine;

(3) Removal of protecting groups; and

(4) Modifications of an existing N-prodrug of gemcitabine.

Protection and deprotection in the Schemes may be carried out at various stages of the synthesis according to the procedures generally known in the art (e.g., "Protecting Groups in Organic Synthesis," 3rd Edition, Wiley, 1999).

The following exemplifies a method for preparing a particular compound of present invention, i.e., 4-((4-(3-chlorophenyl)-2-oxido-1,3,2-dioxaphosphinan-2-yl)amino)-1-((2R,4R,5R)-3,3-difluoro-4-hydroxy-5-(hydroxymethyl)tetrahydrofuran-2-yl)pyrimidin-2(1H)-one.

(1) Protection of the 5'-position of gemcitabine

Selective protection of the 3',5'-OH group of nucleosides are often achievable under suitable reaction conditions. For example, treatment of gemcitabine with tert-butyldimethylsilyl chloride (TBSCl) in the presence of a suitable base and suitable reaction conditions produces 3',5'-OTBS derivative of gemcitabine shown as follows.

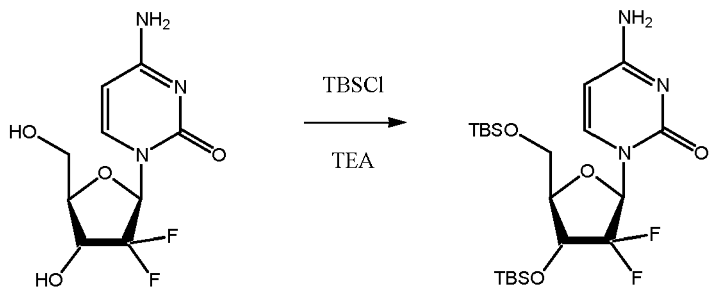


1 2

(2) Preparation of N-prodrugs of Gemcitabine

Prodrugs can be introduced at different stages of the synthesis. Most often these prodrugs are introduced at the later stage of synthesis due to the lability of various prodrugs, while prodrugs could also be introduced at an early stage of the synthesis due to other considerations. For example, treatment of 3',5'-TBS protected gemcitabine with a para-nitrophenol 1,3-propanediol cyclic phosphate ester (Q) in the presence of a suitable base such as sodium hydride produces the N-phosphate ester prodrug derivative shown as follows. Alternatively, a racemic version of compound Q could also be used to produce compound 3 as diastereoisomers at the benzylic chiral center, and later separate out the two diastereoisomers.

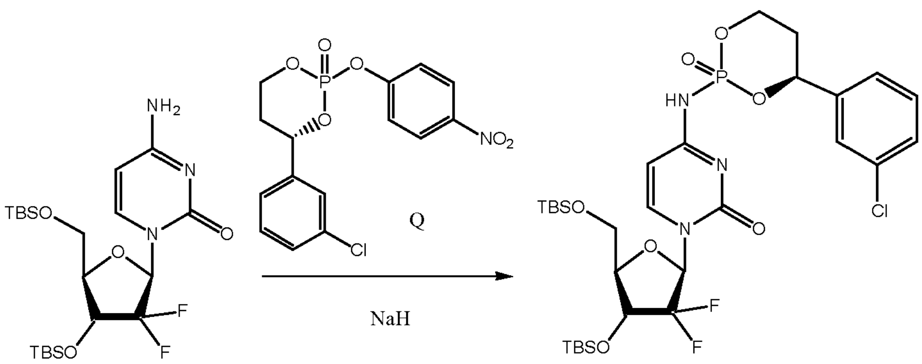


Q

2 3

Other phosphorylation reagents and methods could also be used to attach a phosphorus-containing group to the 4-amino group of either a suitably protected form of gemcitabine or gemcitabine itself directly.

(3) Removal of protecting groups

Once the desired N-prodrug group is attached, the molecule could be either further modified at other positions or undergo deprotection reaction to remove protecting groups. For example, treatment of compound X with ammonium fluoride under suitable reaction conditions removes the 3',5'-TBS protecting groups.

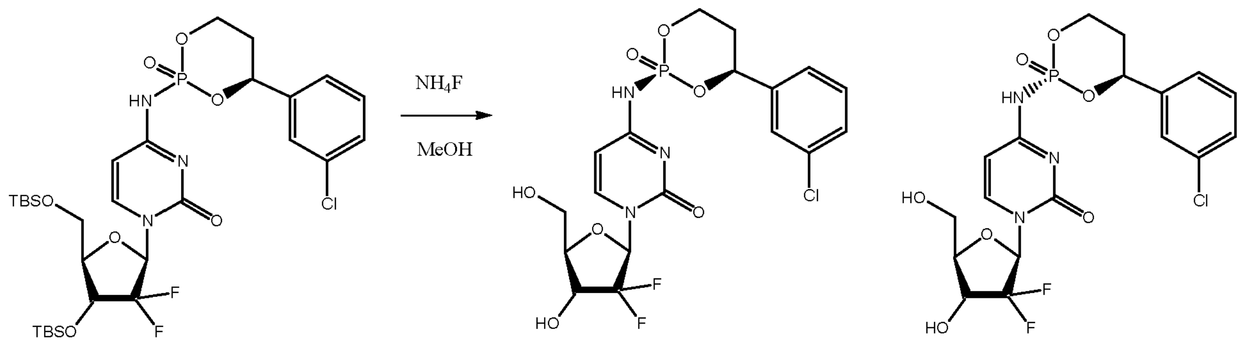


3 4 5

(4) Modifications of an existing N-prodrug of gemcitabine

When necessary, other modifications could also be made at other positions of gemcitabine. Alternatively, another phosphate prodrug group could be attached to the 5'-position selectively. For example, treatment of compound X with phenyl dichlorophosphate followed by treatment with alanine ethyl ester in the presence of a suitable base under suitable reaction condition leads to compound Y wherein both 5'- and N4-positions have phosphate prodrugs.

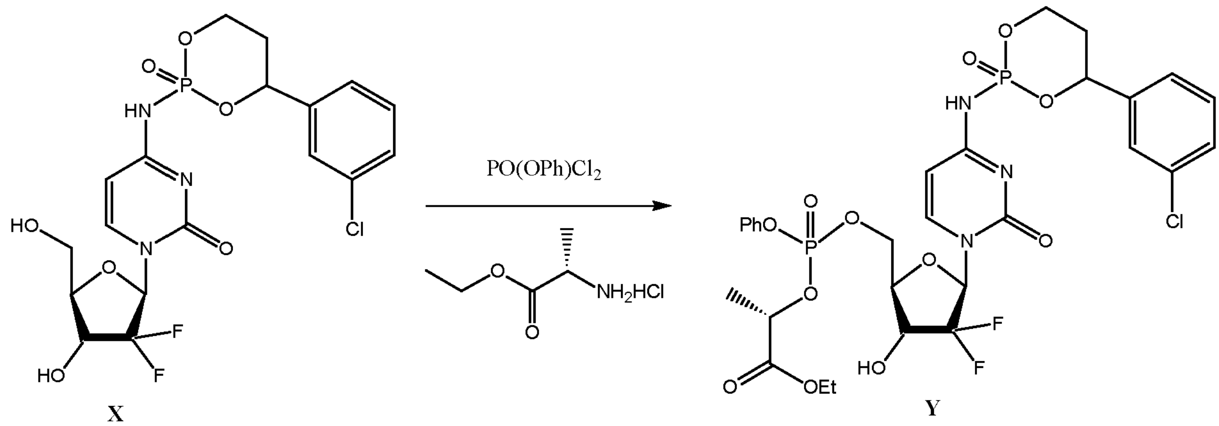


X Y

In another aspect, a cyclic group could be formed, linking the 3' and 5'-OH groups. For example, treatment of compound X with phosphoryl trichloride followed by treatment with alanine ethyl ester in the presence of a suitable base and under suitable reaction conditions generate compounds of formula I wherein $R^3$ and $R^5$ together formed a cyclic phosphate group.

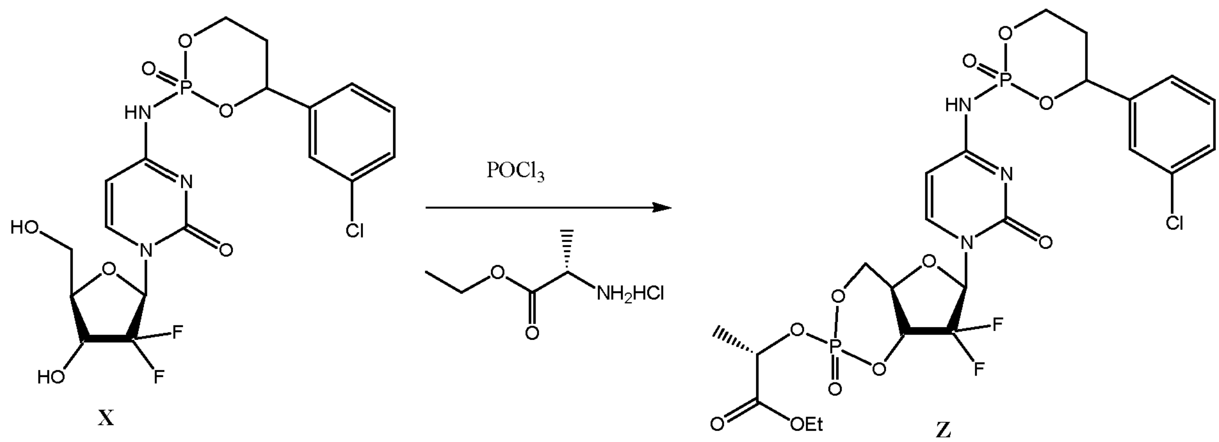


X Z

**Preparation of key precursors**

Various precursors could be prepared according to procedures reported by prior art documents. For example,

the nitrophenyl 1,3-propane diol cyclic phosphate ester could be prepared following the procedures disclosed in J. Am. Chem. Soc., 2004, 126, 5154-5163.

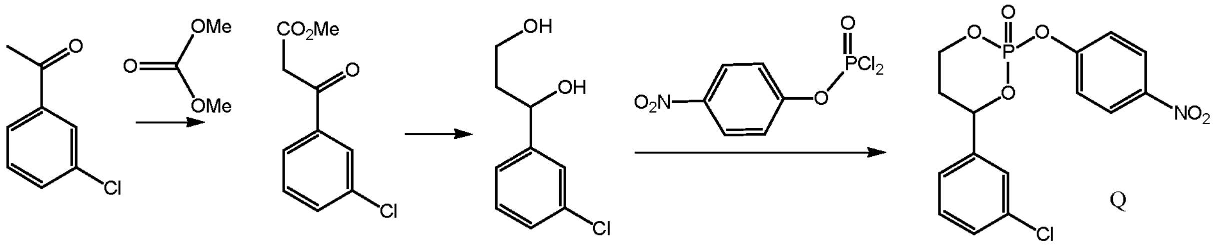

Q

Alternatively, the propane-1,3-diol could be prepared stereoselectively to give a chiral compound and a chiral compound Q could be obtained after reacting with dichlorophosphoryl p-nitrophenol ester.

Other precursors of formula (V) can be prepared in a similar manner as above.

All stereoisomers of the compounds of the instant invention are contemplated, either in admixture or in pure or substantially pure form. The compounds of the present invention can have stereogenic centers at the phosphorus atom and at any of the carbons including any of the R substituents. Consequently, compounds of formula I can exist in enantiomeric or diastereomeric forms or in mixtures thereof. The processes for preparation can utilize racemates, enantiomers or diastereomers as starting materials. When enantiomeric or diastereomeric products are prepared, they can be separated by conventional methods. For example, chromatography or fractional crystallization can be used to separate diastereomeric mixtures, while derivatives of enantiomeric isomers can be separated via chromatography.

One aspect of the present invention provides a method to synthesize and isolate single isomers of compounds of Formula I. Because phosphorus is a stereogenic atom, formation of a prodrug with a racemic substituted 1,3-propane diol will produce a mixture of isomers. In another aspect, the use of the enantioenriched substituted 1,3-propane diol with the R configuration gives enantioenriched R cis and R trans prodrugs. These compounds can be separated by a combination of column chromatography and/or fractional crystallization.

Prodrugs often are introduced at the later stage of synthesis, while some prodrugs could also be introduced at an early stage of the synthesis due to other considerations.

Alternatively, the compounds of formula I can be prepared by using suitable protecting groups to block the 3' and 5' hydroxyl functions followed by functionalization of the N4-amino group. Typical protecting groups are well known and summarized in the art (Protecting Groups in Organic Synthesis, 3rd edition Theodora Greene, Peter Wuts (Wiley-Interscience) 1999). On the other hand, the compounds of formula I may be prepared without the use of protecting groups.

## Formulations, dosage and use

Compounds of the invention are administered in a total daily dose of 0.01 to 2500 mg. In one aspect the range is about 5 mg to about 500 mg. The dose may be administered in as many divided doses as is convenient.

Compounds of this invention when used in combination with other agents may be administered as a daily dose or an appropriate fraction of the daily dose (e.g., bid). The compounds of this invention may be used in a multidrug regimen, also known as combination or 'cocktail' therapy, wherein, multiple agents may be administered together, may be administered separately at the same time or at different intervals, or administered sequentially. The compounds of this invention may be administered after a course of treatment by another agent, during a course of therapy with another agent, administered as part of a therapeutic regimen, or may be administered prior to therapy by another agent in a treatment program.

For the purposes of this invention, the compounds may be administered by a variety of means including orally, parenterally, by inhalation spray, topically, or rectally in formulations containing pharmaceutically acceptable carriers, adjuvants and vehicles. The term parenteral as used here includes subcutaneous, intravenous, intramuscular, and intraarterial injections with a variety of infusion techniques. Intraarterial and intravenous injection as used herein includes administration through catheters. Intravenous administration is generally preferred.

Pharmaceutically acceptable salts include acetate, adipate, besylate, bromide, camsylate, chloride, citrate, edisylate, estolate, fumarate, gluceptate, gluconate, glucoranate, hippurate, hyclate, hydrobromide, hydrochloride, iodide, isethionate, lactate, lactobionate, maleate, mesylate, methylbromide, methylsulfate, napsylate, nitrate, oleate, palmoate, phosphate, polygalacturonate, stearate, succinate, sulfate, sulfosalicylate, tannate, tartrate, terphthalate, tosylate, and triethiodide.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions

intended for oral use may be prepared according to any method known in the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, ethylcellulose, hydroxypropylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n propyl p hydroxy benzoate, one or more coloring agents, one or more flavoring agents and one or more sweetening agents, such as sucrose or saccharin.

Oil suspensions may be formulated by suspending the active ingredient in a vegetable oil, such as arachid oil, olive oil, sesame oil or coconut oil, or in a mineral oil such as liquid paraffin. The oral suspensions may contain a thickening agent, such as beeswax, hard paraffin or acetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil in water emulsions. The oily phase may be a vegetable oil, such as olive oil or arachid oil, a mineral oil, such as liquid paraffin, or a mixture of these. Suitable emulsifying agents include naturally occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butanediol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose, any bland fixed oil may be employed including synthetic mono or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time release formulation intended for oral administration to humans may contain 20 to 2000 μmol (approximately 10 to 1000 mg) of active material compounded with an appropriate and convenient amount of carrier material which may vary from about 5 to about 95% of the total compositions. It is preferred that the pharmaceutical composition be prepared which provides easily measurable amounts for administration. For example, an aqueous solution intended for intravenous infusion should contain from about 0.05 to about 50 μmol (approximately 0.025 to 25 mg) of the active ingredient per milliliter of solution in order that infusion of a suitable volume at a rate of about 30 mL/hr can occur.

As noted above, formulations of the present invention suitable for oral administration may be presented as

discrete units such as capsules, cachets or tablets each containing a predetermined amount of the active ingredient; as a powder or granules; as a solution or a suspension in an aqueous or non-aqueous liquid; or as an oil in water liquid emulsion or a water in oil liquid emulsion. The active ingredient may also be administered as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross linked povidone, cross linked sodium carboxymethyl cellulose) surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropyl methylcellulose in varying proportions to provide the desired release profile. Tablets may optionally be provided with an enteric coating, to provide release in parts of the gut other than the stomach. This is particularly advantageous with the compounds of Formula I when such compounds are susceptible to acid hydrolysis.

Formulations suitable for topical administration in the mouth include lozenges comprising the active ingredient in a flavored base, usually sucrose and acacia or tragacanth; pastilles comprising the active ingredient in an inert base such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit dose or multi dose sealed containers, for example, ampoules and vials, and may be stored in a freeze dried (lyophilized) condition requiring only the addition of the sterile liquid carrier, for example water for injections, immediately prior to use. Injection solutions and suspensions may be prepared from sterile powders, granules and tablets of the kind previously described.

Formulations suitable for parenteral administration may be administered in a continuous infusion manner via an indwelling pump or via a hospital bag. Continuous infusion includes the infusion by an external pump. The infusions may be done through a Hickman or PICC or any other suitable means of administering a formulation either parenterally or i.v.

Preferred unit dosage formulations are those containing a daily dose or unit, daily sub dose, or an appropriate fraction thereof, of a drug.

It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those skilled in the art.

The phosphorus-containing prodrugs of gemcitabine according to present invention could be converted into gemcitabine *in vivo* and thus can be used for the treatment of susceptible neoplasms, wherein the susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC). In this regard, the prodrugs of present invention could also be used in combination with one or more other active agents/methods for the treatment of cancers, such as antiproliferative/antineoplastic drugs, cytostatic agents, anti-invasion agents, growth factor inhibitors, antiangiogenic agents, gene therapy approaches, immunotherapy approaches, cytotoxic agents, and target therapies (for example PI3Kd inhibitors).

## Examples

The compounds used in this invention and their preparation can be understood further by the Examples, which illustrate some of the processes by which these compounds are prepared. These Examples should not however be construed as specifically limiting the invention, and variations of the compounds, now known or later developed, are considered to fall within the scope of the present invention as hereinafter claimed. Unless otherwise indicated, the starting materials and reagents used in Examples were commercially available (such as from Aldrich) or prepared according to known methods described in prior art documents.

The following abbreviations are used in this specification:

DMAP - dimethylaminopyridine

DMF - dimethylformamide

DIEA - diisopropylethylamine

TEA - triethylamine

TBSCl - tert-butyldimethylsilyl chloride

EtOAc - ethyl acetate

THF - tetrahydrofuran

TBAF - tetra-butylammonium fluoride

NMR - nuclear magnetic resonance

LC-MS - liquid chromatography mass spectrometry

TMS - tetramethylsilane

RT - room temperature

i.v. - intravenous administration

PO - oral administration

THU- tetrahydrouridine

PK- Pharmacokinetics

$MRT_{0-t}$ - Mean residence time from time zero to time observed

$MRT_{0-inf}$ - Mean residence time from time zero to infinity

$AUC_{0-t}$ - Area under the curve from time zero to time observed

$AUC_{0-inf}$- Area under the curve from time zero to infinity

TI -Therapeutic Index

**General experimental methods**

$^1H$ NMR spectra were recorded on Bruker Avance III and Bruker Avance Neo, 400 MHz and TMS was used as an internal standard.

LC-MS was taken on a quadrupole Mass Spectrometer on Agilent LC/MSD 1200 Series (Column: Ultimate XB-C18 (50 × 4.6 mm, 5 μm) operating in ES (+) or (-) ionization mode; T = 30 °C; flow rate = 1.5 mL/min; detection wavelength: 214 nm.

**Example 1**

**Preparation of 4-(((2R,4S)-4-(3-chlorophenyl)-2-oxido-1,3,2-dioxaphosphinan-2-yl)amino)-1-((2R,4R,SR)-3,3-difluoro-4-hydroxy-5-(hydroxymethyl)tetrahydrofuran-2-yl)pyrimidin-2(1H)-one (compound 4) & 4-(((2S,4S)-4-(3-chlorophenyl)-2-oxido-1,3,2-dioxaphosphinan-2-yl)amino)-1-((2R,4R,SR)-3,3-difluoro-4-hydroxy-5-(hydroxymethyl)tetrahydrofuran-2-yl)pyrimidin-2(1H)-one (compound 4a)**

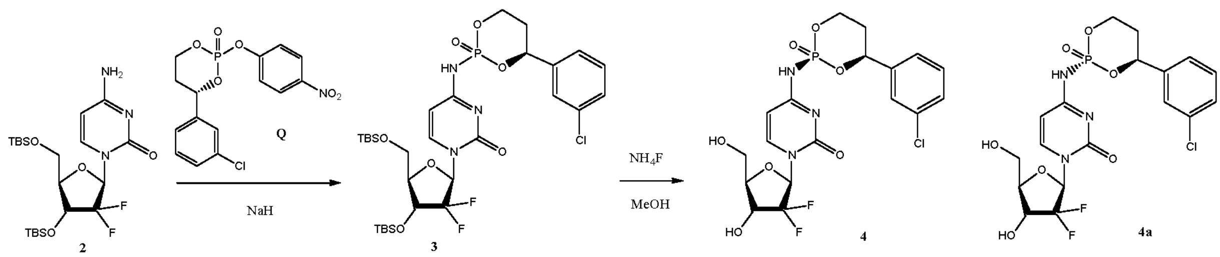


(i) Preparation of 1-((2R,4R,5R)-4-((tert-butyldimethylsilyl)oxy)-5-(((tert-butyldimethylsilyl)oxy)methyl)-3,3-difluorotetrahydrofuran-2-yl)-4-(((4S)-4-(3-chlorophenyl)-2-oxido-1,3,2-dioxaphosphinan-2-yl)amino)pyrimidin-2(1H)-one (compound 3)

To a solution of 4-amino-1-((2R,4R,5R)-4-((tert-butyldimethylsilyl)oxy)-5-(((tert-butyldimethylsilyl)-oxy)methyl)-3,3-difluorotetrahydrofuran-2-yl)pyrimidin-2(1H)-one (compound **2**, 1.0 g, 2.04 mmol) in THF (15.0 mL) was added NaH (122 mg, 3.18 mmol, 60% in mineral oil) portionwisely at 0 °C. After addition, the mixture was stirred at the same temperature for 1 hour. Then (4S)-4-(3-chlorophenyl)-2-(4-nitrophenoxy)-1,3,2-dioxaphosphinane 2-oxide (**Q,** 752 mg, 2.04 mmol; prepared according to the procedures of Journal of Medicinal Chemistry, 2018, vol 61, page 4904) in THF (5.0 mL) was added dropwise into above mixture at 0 °C. Then the reaction mixture was stirred at 0 °C for another 2 hours. The reaction was quenched with $H_2O$ (30 mL), then extracted with EtOAc (50 mL*2). The combined organic layers were washed with brine (50 mL), dried over $Na_2SO_4$, filtered and concentrated. The residue was purified by column chromatography on silica gel (DCM/$CH_3OH$ = 80/1) to give the desired product (compound **3**, 620 mg, 42%) as a yellow solid.

LC-MS: Rt = 1.93 min, $[M+H]^+$ = 722.

(ii) Preparation of 4-(((2R,4S)-4-(3-chlorophenyl)-2-oxido-1,3,2-dioxaphosphinan-2-yl)amino)-1-((2R,4F,5R)-3,3-difluoro-4-hydroxy-5-(hydroxymethyl)tetrahydrofuran-2-yl)pyrimidin-2(1H)-one (compound **4**) & 4-(((2S,4S)-4-(3-chlorophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one (compound **4a**)

To a solution of 1-((2R,4R,SR)-4-((tert-butyldimethylsilyl)oxy)-5-(((tert-butyldimethylsilyl)-oxy)methyl)-3,3-difluorotetrahydrofuran-2-yl)-4-(((4S)-4-(3-chlorophenyl)-2-oxido-1,3,2-dioxaphosphinan-2-yl)amino)pyrimidin-2(1H)-one (compound **3**, 620 mg, 0.86 mol, 5) in $CH_3OH$ (10 mL) was added $NH_4F$ (318 mg, 8.6 mmol) and the mixture was stirred at 25 °C for 24 hrs. The mixture was purified by Prep-HPLC to afford the product compound 4 (112 mg) and compound **4a** (33mg) as white solids.

Compound **4**: 1H NMR (400 MHz, DMSO-d6): δ 11.0 (brs, 1H), 7.91 (d, J = 8.8 Hz, 1H), 7.50 (s, 1H), 7.43-7.38 (m, 3H), 6.33-6.27 (m, 2H), 6.12 (t, J = 7.6 Hz, 1H), 5.65 (d, J = 10.8 Hz, 1H), 5.25 (s, 1H), 4.56-4.50 (m, 1H), 4.40-4.35 (m, 1H), 4.19-4.15 (m, 1H), 3.88-3.84 (m, 1H), 3.78 (d, J = 12.4 Hz, 1H), 3.63 (d, J = 12.4 Hz, 1H), 2.48-2.31 (m, 1H), 2.08-2.03 (m, 1H).

LC-MS: Rt = 2.863 min, $[M+H]^+$ = 494.

Compound **4a**: 1H NMR (400 MHz, DMSO-d6): δ 11.7 (brs, 1H), 7.89 (d, J = 7.6 Hz, 1H), 7.46-7.36 (m, 4H), 6.31 (d, J = 5.6 Hz, 1H), 6.24 (d, J = 7.6 Hz, 1H), 6.10 (t, J = 8.0 Hz, 1H), 5.56 (brs, 1H), 5.24 (s, 1H), 4.48-4.33 (m, 2H), 4.19-4.15 (m, 1H), 3.87-3.84 (m, 1H), 3.77 (d, J = 12.0 Hz, 1H), 3.63 (d, J = 12.0 Hz, 1H), 2.10-2.00 (m, 2H).

LC-MS: Rt = 2.954 min, $[M+H]^+$ = 494.

**Example 2**

**Synthesis of 4-amino-1-((2R,3S,4S,SR)-3,4-dihydroxy-5-(hydroxymethyl)tetrahydrofuran-2-yl)pyrimidin-2(1H)-one (Compound 12)**

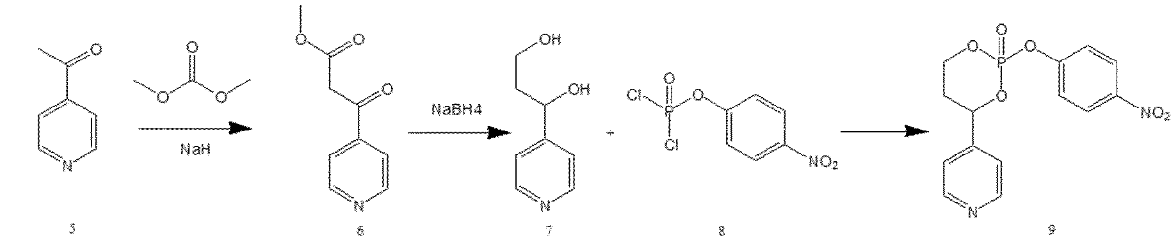

5 6 7 8 9

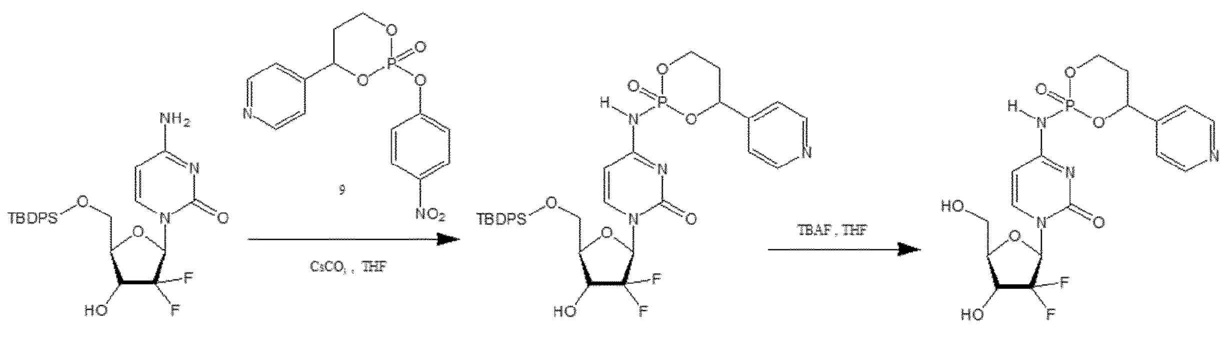

10 11 12

**Synthesis of methyl 3-oxo-3-(pyridin-4-yl)propanoate (Compound 6)**

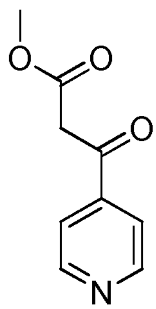

To a mixture of NaH (6.6 g, 165.2 mmol, 60% in mineral oil) in toluene (120 mL) was added dimethyl carbonate (11.2 g, 123.9 mmol) in toluene (40 mL). Then 1-(pyridin-4-yl)ethanone (5.0 g, 41.3 mmol, **5**) in toluene (40 mL) was added dropwise under $N_2$. The reaction mixture was stirred at 105 °C for 12 hours. After cooled to RT, the reaction was quenched with 200 mL (water) and HOAc (20 mL) and the mixture was stirred at RT for 20 minutes. Then the mixture was extracted with EtOAc (300 mL*3). The combined organic layer was dried over $Na_2SO_4$, filtered and concentrated. The residue was purified by Prep-HPLC (water/acetonitrile = 10% to 90%) to give the title product (3.89 g, 53%, **6**) as a brown solid.

LC-MS: Rt = 1.20 min, $[M+H]^+$ = 180.

**Synthesis of 1-(pyridin-4-yl)propane-1,3-diol (Compound 7)**

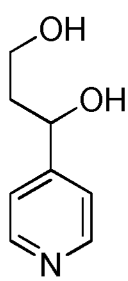

To a mixture of methyl 3-oxo-3-(pyridin-4-yl)propanoate (3.89 g, 21.7 mmol, **6**) in MeOH (80 mL) was added $NaBH_4$ (6.60 g, 173.6 mmol) portion-wise at 0 °C. The mixture was then stirred at 70 °C for 16 hours under $N_2$. The reaction mixture was concentrated. The residue was purified by Prep-HPLC (water/acetonitrile = 2% to 80%) to give the title compound (3.21 g, 96%, 7) as a yellow solid.

LC-MS: Rt = 0.28 min, $[M+H]^+$ = 154.

**Synthesis of 2-(4-nitrophenoxy)-4-(pyridin-4-yl)-1,3,2-dioxaphosphinane 2-oxide (Compound 9)**

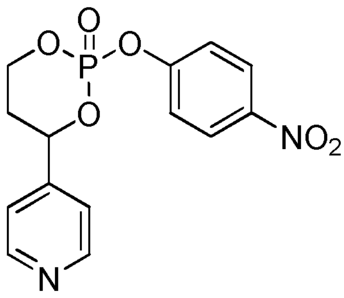

A mixture of 4-nitrophenyl phosphorodichloridate (5.89 g, 23.01 mmol,**8**) in THF (60 mL) was added dropwise to a mixture of 1-(pyridin-4-yl)propane-1,3-diol (3.2 g, 20.92 mmol, **7**) and TEA (8.45 g, 83.66 mmol) in THF (100 mL) at RT, then stirred at 75 °C for 4 hours. Then to above mixture was added TEA (8.45 g, 83.66 mmol), followed by addition of 4-nitro-phenol (11.63 g, 83.66 mmol) in THF (80 mL) slowly at RT. The reaction mixture was stirred at RT overnight. The reaction mixture was treated with ethyl acetate (200 mL), washed with 0.4M NaOH (80 mL*4), brine (100 mL*3). The organic layer was dried over anhydrous $Na_2SO_4$ and concentrated. The residue was purified by silica gel column (petroleum ether:EtOAc = 1:4) to afford the title compound (1.85 g, 26%, **9**) as a yellow solid.

LC-MS: Rt = 1.36 min, $[M+H]^+$ = 337.

**Synthesis of 1-((2R,4R,SR)-5-(((tert-butyldiphenylsilyl)oxy)methyl)-3,3-difluoro-4-hydroxytetrahydrofuran-2-yl)-4-((2-oxido-4-(pyridin-4-yl)-1,3,2-dioxaphosphinan-2-yl)amino)pyrimidin-2(1H)-one (Compound 11)**

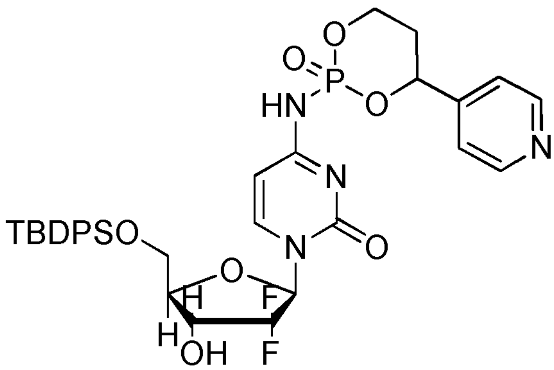

To a mixture of 4-amino-1-((2R,4R,5R)-5-(((tert-butyldiphenylsilyl)oxy)methyl)-3,3-difluoro-4-hydroxytetrahydrofuran-2-yl)pyrimidin-2(1H)-one (400 mg, 0.80 mmol,**10**) and $Cs_2CO_3$ (522 mg, 1.60 mmol) in THF (10 mL) was added 2-(4-nitrophenoxy)-4-(pyridin-4-yl)-1,3,2-dioxaphosphinane 2-oxide (269 mg, 0.80 mmol, **9**) at room temperature. After addition, the reaction mixture was stirred at 30 °C for 16 hours. Then the reaction mixture was quenched with water (15 mL), extracted with EtOAc (15 mL * 2). The combined organic phase was washed with brine (15 mL), dried over $Na_2SO_4$, filtered and concentrated in vacuo. The residue was purified by C18 column (water/acetonitrile = 20% to 80%) to give compound **11** (450 mg, 81%) as a white solid.

LC-MS: Rt = 1.649 min, $[M+H]^+$ = 699.

**Synthesis of 1-((2R,4R,SR)-3,3-difluoro-4-hydroxy-5-(hydroxymethyl)tetrahydrofuran-2-yl)-4-((2-oxido-4-(pyridin-4-yl)-1,3,2-dioxaphosphinan-2-yl)amino)pyrimidin-2(1H)-one (Compound 12)**

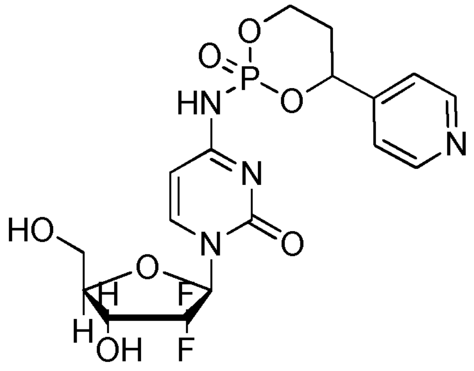

To a solution of 1-((2R,4R,5R)-5-(((tert-butyldiphenylsilyl)oxy)methyl)-3,3-difluoro-4-hydroxytetrahydrofuran-2-yl)-4-((2-oxido-4-(pyridin-4-yl)-1,3,2-dioxaphosphinan-2-yl)amino)pyrimidin-2(1H)-one (450 mg, 0.64 mmol,11) in THF (4 mL) was added TBAF (1.28 mL, 1.28 mmol, 1 M in THF) dropwise at 0°C. After addition, the reaction mixture was stirred at room temperature for 1 hour. The reaction mixture was purified by C18 column (water/acetonitrile= 10% to 50%) to give Compound **12** (64 mg, 22%) as a white solid.

$^1$H NMR (400MHz, DMSO-$d_6$): δ 8.61-8.57 (m, 2H), 7.83-7.79 (m, 1H), 7.55-7.48 (m, 2H), 6.33-6.29 (m, 1H), 5.94-5.91 (m, 1H), 5.81-5.72 (m, 1H), 5.15-5.09 (m, 1H), 4.73-4.56 (m, 2H), 4.26-4.22 (m, 1H), 4.03-3.84 (m, 2H), 2.29-2.26 (m, 2H).

LC-MS: $Rt_1$ = 3.078, $Rt_2$ = 3.227, $Rt_3$ = 3.296, $Rt_4$ = 3.465 min; $[M+H]^+$ = 461

**Example 3**

**Synthesis of 4-amino-1-((2R,3S,4S,SR)-3,4-dihydroxy-5-(hydroxymethyl)tetrahydrofuran-2-yl)pyrimidin-2(1H)-one (Compound 18)**

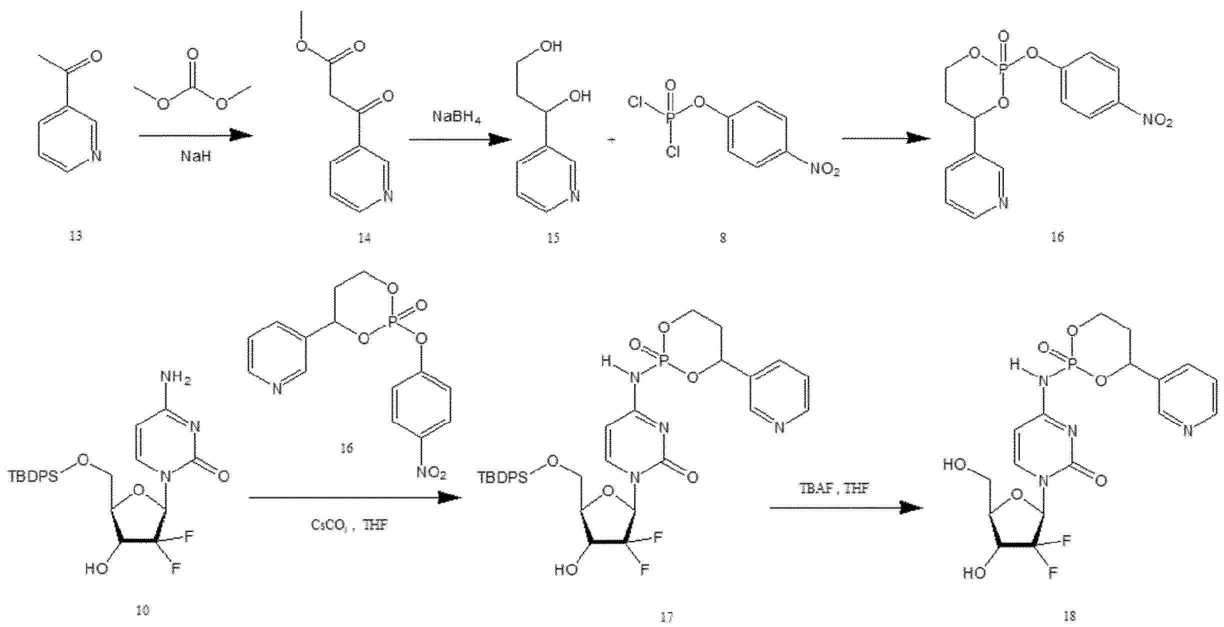


13 14 15 8 16

10 17 18

**Synthesis of methyl 3-oxo-3-(pyridin-3-yl)propanoate (Compound 14)**

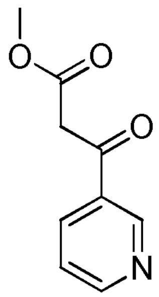

To a mixture of NaH (6.4 g, 160 mmol, 60% in mineral oil) in toluene (120 mL) was added dimethyl carbonate (10.8 g, 120 mmol) in toluene (40 mL). Then 1-(pyridin-3-yl)ethanone (5.0 g, 41.3 mmol, **13**) in toluene (40 mL) was added dropwise under $N_2$. The reaction mixture was stirred at 105 °C for 12 hours. After cooled to RT, the reaction was quenched with 200 mL (water) and HOAc (20 mL). Then the mixture was extracted with EtOAc (100 mL*4). The combined organic layer was dried over $Na_2SO_4$, filtered and concentrated. The residue was purified by Prep-HPLC (water/acetonitrile = 10% to 90%) to give the title product (4.5 g, 60.8%, **14**) as a red solid.
LC-MS: Rt = 1.15 min, $[M+H]^+$ = 180.

**Synthesis of 1-(pyridin-3-yl)propane-1,3-diol (Compound 15)**

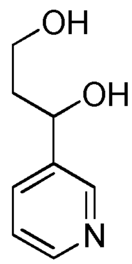

To a stirred mixture of methyl 3-oxo-3-(pyridin-3-yl)propanoate (4.0 g, 22.3 mmol, **14**) in MeOH (50 mL) was added $NaBH_4$ (6.80 g, 178.9 mmol) portion-wise at 0 °C. The mixture was then stirred at 70 °C for 16 hours under $N_2$. The reaction mixture was concentrated. The residue was purified by Prep-HPLC (water/acetonitrile = 2% to 80%) to give the title compound (3.21 g, 96%, **15**) as a yellow oil.
LC-MS: Rt = 0.32 min, $[M+H]^+$ = 154.

**Synthesis of 2-(4-nitrophenoxy)-4-(pyridin-3-yl)-1,3,2-dioxaphosphinane 2-oxide (Compound 16)**

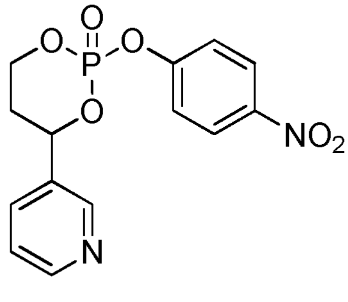

To a solution of 4-nitrophenyl phosphorodichloridate (14.4 g, 56.2 mmol, **8**) in THF (80 mL) was added 1-(pyridin-3-yl)propane-1,3-diol (4.3 g, 28.1 mmol, **15**) and TEA (11.35 g, 112.4 mmol) in THF (20 mL) at 10 °C. The reaction mixture was stirred at 75 °C under $N_2$ for 2 hours. Then TEA (11.35 g, 112.4 mmol) was added after the mixture was cooled to RT naturally, followed by addition of 4-nitro-phenol (15.6 g, 112.4 mmol) in THF (50 mL) slowly. The mixture was stirred at RT for another 16 hours. The reaction mixture was diluted with $H_2O$ (400 mL), then extracted with EtOAc (100 mL*3). The combined organic layers were dried over $Na_2SO_4$, filtered and concentrated. The residue was purified by silica gel column (petroleum ether/EtOAc = 3/1 to EtOAc) to give the title product (0.97 g, 10.4%,**16**) as a yellow oil.

LC-MS: Rt = 1.36 min, $[M+H]^+$ = 337.

**Synthesis of 1-((2R,4R,SR)-5-(((tert-butyldiphenylsilyl)oxy)methyl)-3,3-difluoro-4-hydroxytetrahydrofuran-2-yl)-4-((2-oxido-4-(pyridin-3-yl)-1,3,2-dioxaphosphinan-2-yl)amino)pyrimidin-2(1H)-one (Compound 17)**

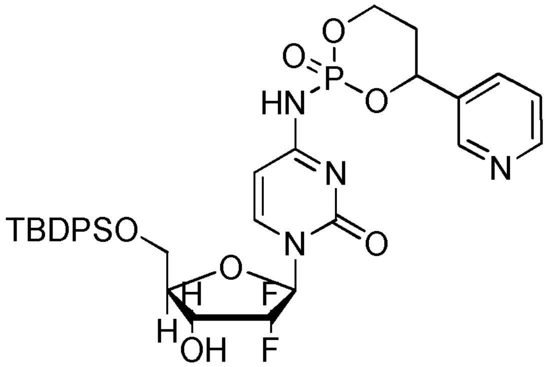

To a mixture of 4-amino-1-((2R,4R,5R)-5-(((tert-butyldiphenylsilyl)oxy)methyl)-3,3-difluoro-4-hydroxytetrahydrofuran-2-yl)pyrimidin-2(1H)-one (400 mg, 0.80 mmol, **10**) and $Cs_2CO_3$ (522 mg, 1.60 mmol) in THF (10 mL) was added 2-(4-nitrophenoxy)-4-(pyridin-3-yl)-1,3,2-dioxaphosphinane 2-oxide (269 mg, 0.80 mmol, **16**) at room temperature. After addition, the reaction mixture was stirred at 30 °C for 16 hours. Then the reaction mixture was quenched with water (20 mL), extracted with EtOAc (30 mL*3). The combined organic phase was concentrated in vacuo. The residue was purified by C18 column (water/acetonitrile=20% to 70%) to give title compound (430 mg, 77%, **17**) as a white solid.

LC-MS: Rt = 1.711 min, $[M+H]^+$ = 699.

**Synthesis of 1-((2R,4R,SR)-3,3-difluoro-4-hydroxy-5-(hydroxymethyl)tetrahydrofuran-2-yl)-4-((2-oxido-4-(pyridin-3-yl)-1,3,2-dioxaphosphinan-2-yl)amino)pyrimidin-2(1H)-one (Compound 18)**

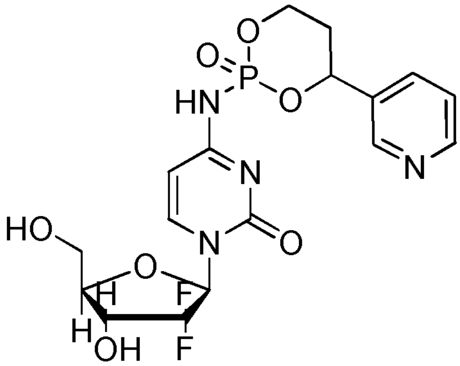

To a solution of 1-((2R,4R,SR)-5-(((tert-butyldiphenylsilyl)oxy)methyl)-3,3-difluoro-4-hydroxytetrahydrofuran-2-yl)-4-((2-oxido-4-(pyridin-3-yl)-1,3,2-dioxaphosphinan-2-yl)amino)pyrimidin-2(1H)-one (430 mg, 0.62 mmol) in THF (4 mL) was added TBAF (1.24 mL, 1.24 mmol, 1 M in THF) dropwise at 0 °C. After addition, the reaction mixture was stirred at room temperature for 1 hour. The reaction mixture was purified by C18 column (water/acetonitrile= 10% to 50%) to give Compound **18** (34 mg, 12%) as a white solid.

$^1H$ NMR (400MHz, DMSO-$d_6$): δ 8.65-8.55 (m, 2H), 7.98-7.93 (m, 1H), 7.83-7.79 (m, 1H), 7.53-7.49 (m, 1H),

6.36-6.31 (m, 1H), 5.94-5.91 (m, 1H), 5.82-5.75 (m, 1H), 5.20-5.07 (m, 1H), 4.76-4.56 (m, 2H), 4.26-4.23 (m, 1H), 4.05-3.81 (m, 2H), 2.40-2.21(m, 2H).

LC-MS: $Rt_1$ = 3.164, $Rt_2$ = 3.372, $Rt_3$ = 3.521, $[M+H]^+$ = 461.

In a similar manner, the following compounds of formula I wherein Ar is an aryl group are prepared:

4-((4-(3-bromophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;

1-((2R,4R,5R)-3,3-difluoro-4-hydroxy-5-(hydroxymethyl)tetrahydrofuran-2-yl)-4-((4-(3-fluorophenyl)-2-oxido-1,3,2-dioxaphosphinan-2-yl)amino)pyrimidin-2(1H)-one;

1-((2R,4R,5R)-3,3-difluoro-4-hydroxy-5-(hydroxymethyl)tetrahydrofuran-2-yl)-4-((4-(4-methoxyphenyl)-2-oxido-1,3,2-dioxaphosphinan-2-yl)amino)pyrimidin-2(1H)-one; and

((2R,3R,5R)-5-(4-((4-(3-chloro-4-methoxyphenyl)-2-oxido-1,3,2-dioxaphosphinan-2-yl)amino)-2-oxopyrimidin-1(2H)-yl)-4,4-difluoro-3-hydroxytetrahydrofuran-2-yl)methyl pivalate.

**Example 4**

**Synthesis of (2R,3R,SR)-5-(4-((4-(3-chlorophenyl)-2-oxido-1,3,2-dioxaphosphinan-2-yl)amino)-2-oxopyrimidin-1(2H)-yl)-4,4-difluoro-2-((isobutyryloxy)methyl)tetrahydrofuran-3-yl isobutyrate (Compound 19)**

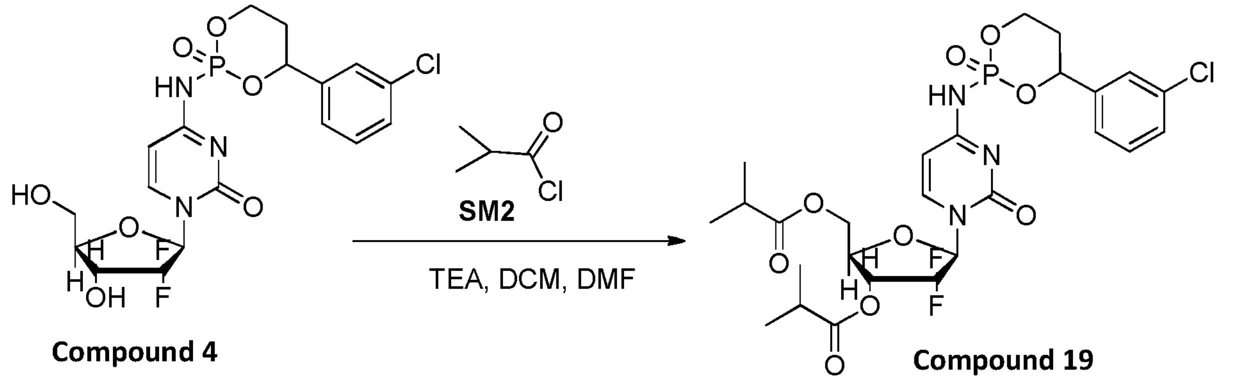


Compound 4

Compound 19

To a mixture 4-((4-(3-chlorophenyl)-2-oxido-1,3,2-dioxaphosphinan-2-yl)amino)-1-((2R,4R,5R)-3,3-difluoro-4-hydroxy-5-(hydroxymethyl)tetrahydrofuran-2-yl)pyrimidin-2(1H)-one (150 mg, 0.304 mmol, Compound **4**, prepared according to Example 1) in DCM/DMF (3 mL/0.3 mL) was added TEA (154 mg, 1.52 mmol), followed by the addition of isobutyryl chloride (324 mg, 3.04 mmol, SM2) dropwise at RT. After addition, the reaction mixture was stirred at RT for 16 hours. The mixture was quenched with water (50 mL), extracted with EtOAc (20 mL*2). The combined organic phase was concentrated. The residue was purified by C18 (water/acetonitrile= 10% to 60%) to give the title compound (41 mg, Compound **19**) as a white solid. The white solid (Y1430-12483-022 (49 mg) and Y1430-12483-034 (41 mg)) was combined (90 mg, total yield 21%, Compound **19** as a mixture of diastereo-isomers).

$^1$H NMR (400MHz, DMSO-$d_6$): δ 8.01-7.98 (m, 1H), 7.54-7.34 (m, 5H), 6.36-6.34 (m, 1H), 5.73-5.64 (m, 1H), 5.19-5.15 (m, 1H), 4.72-4.46 (m, 5H), 2.72-2.57 (m, 2H), 2.36-2.16 (m, 2H), 1.23-1.09 (m, 12H).

LC-MS: $R_{t1}$ = 2.976 min, $R_{t2}$ = 3.165 min, $R_{t3}$ = 3.322 min, $[M+H]^+$ = 634.

In a similar manner the following compounds are prepared:

((2R,3R,5R)-3-acetoxy-5-(4-((4-(3-chlorophenyl)-2-oxido-1,3,2-dioxaphosphinan-2-yl)amino)-2-oxopyrimidin-1(2H)-yl)-4,4-difluorotetrahydrofuran-2-yl)methyl acetate;

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((propionyloxy)methyl)tetrahydrofuran-3-yl propionate;

(2R,3R,5R)-5-(4-((4-(3-chlorophenyl)-2-oxido-1,3,2-dioxaphosphinan-2-yl)amino)-2-oxopyrimidin-1(2H)-yl)-4,4-difluoro-2-((2-methoxyacetoxy)methyl)tetrahydrofuran-3-yl 2-methoxyacetate;

(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((pivaloyloxy)methyl)tetrahydrofuran-3-yl pivalate;

(2R,3R,5R)-5-(4-((4-(3-chlorophenyl)-2-oxido-1,3,2-dioxaphosphinan-2-yl)amino)-2-oxopyrimidin-1(2H)-yl)-4,4-difluoro-2-((isopropyloxycarbonyloxy)methyl)tetrahydrofuran-3-yl isopropyl carbonate.

**Mouse Pharmacokinetic Study**

1. Design of Experiment

1.1 Experimental animal

C57BL/6 mice used in this experiment were purchased from Beijing Vital River Laboratory Animal Technology Co., Ltd., and male mice with a body weight in the range of 16~19 g were tested for administration. The animals were housed with appropriate air conditions (temperature: 20~25 °C; humidity: 40%~70%; light/dark cycle: 12 hours), and were provided with the food and water *ad lib.*

1.2 Formulations

1.2.1 Administration by intravenous injection

The solution media for IV administration were DMSO: 30% solutol HS-15:saline = 10:10:80 (v/v/v). Test substances were dissolved in the media to obtain a clear solution, and filtered through PTFE to get a colorless clear solution for IV administration.

1.2.2 Oral administration

The solution media for oral administration were DMSO: 30% solutol HS-15:saline = 10:10:80 (v/v/v). Test substances were dissolved in the media to obtain a clear solution.

1.3 Administration

The animals were randomly divided into groups. All animals were fasted overnight (12-15 hours) before administration (but provided with water *ad lib*) and provided with foods 4 hours after the administration. As to IV administration of gemcitabine, the mice were administered with the formulations by vena caudalis injection (at dose of 15 mg/kg).

The pharmacokinetic (PK) profile of liver-targeted gemcitabine (dFdC) prodrug according to Formula I (i.e., Compound **4**) was evaluated in male C57BL/C mice following intravenous administration (i.v.) at an equivalent dose to 15 mg/kg of Gemcitabine or following oral administration (PO or i.g.) at a dose of 80 mg/kg.

1.4 Sample collection, processing and analysis

Blood samples of designated animals (n = 3/time point/compound) were taken at 0.083, 0.25, 0.5, 1, 2, 4, and 8 hours following i.v. administration, and at 0.25, 0.5, 1, 2, 4, 8 and 24 hours following PO administration, respectively. Systemic blood samples were collected into EDTA-$K_2$-treated tubes containing tetrahydrouridine (THU, 0.5 mM) to inhibit further metabolism of gemcitabine (dFdC) to dFdU. In addition, at 2 hours following either i.v. or PO administration, 3 animals were sacrificed for collection of liver tissues. Plasma was isolated by centrifugation and frozen prior to analysis. Liver tissue was processed by homogenization in X5 volume of 20% methanol, and the homogenate was subject to centrifugation for collection of the supernatant. Both plasma and tissue concentrations of prodrugs, parent drug (i.e., gemcitabine, aka dFdC) and dFdU (a major metabolite of dFdC) were determined by LC/MS/MS analysis (Instrument: Agilent 6470. MS: Positive Ion, AJS ESI, MRM Detection; HPLC condition: Column: Agilent ZORBAX XDB-C18, 5 $\mu$m, 2.1$\times$50mm (Column No.: 50-241), Flow rate: 0.30 mL·min$^{-1}$). Pharmacokinetic parameters were calculated using WinNonlin software (Pharsight Corp., Mountain View, CA).

2. Results

As can be seen in Figure 1, i.v. or PO administration of the prodrug compound according to Formula I (e.g.

Compound **4**) led to significant exposure of the prodrug in the systemic circulation, suggesting the prodrug was well absorbed in the intestine. Moreover, detection of gemcitabine (aka dFdC) in the circulation further proved that the prodrug approach is viable: dFdC was released from the prodrug *in vivo* (Figure 1).

The prodrug compound according to present invention can enhance dFdC's exposure either by i.v. or oral administration of the prodrug, based on the comparison of the half-life ($T_{1/2}$): $T_{1/2}$=1.1h, 2.1h, and 3.3h for i.v. administration of Gemcitabine, i.v. administration of Compound **4**, and PO administration of Compound **4**, respectively. Other dFdC PK parameters following i.v. administration of Gemcitabine at 15 mg/kg, or i.v. administration (equivalent dose to 15 mg/kg of gemcitabine) of Compound **4**, or PO administration (80 mg/kg) of Compound **4** were shown in Table 1, which demonstrated half-life extension by Compound **4** as compared to Gemcitabine.

Table 1. Plasma PK parameters of dFdC following iv administration of 15 mg/kg Gemcitabine or iv dose of Compound **4** (equivalent dose to 15 mg/kg of gemcitabine), or PO dose of Compound **4** (80mg/kg)

| PK Parameters \ PK Study | | Gemcitabine, i.v. | Compound 4, i.v. | Compound 4, PO |
|---|---|---|---|---|
| $T_{1/2}$ | h | 1.10 | 2.08 | 3.32 |
| $MRT_{0-t}$ | h | 0.46 | 1.29 | 2.42 |
| $MRT_{0-inf}$ | h | 0.51 | 2.62 | 4.40 |
| $AUC_{0-t}$ | $ng \cdot h \cdot mL^{-1}$ | 3270 | 463 | 223 |
| $AUC_{0-inf}$ | $ng \cdot h \cdot mL^{-1}$ | 3290 | 605 | 276 |

Liver drug exposure of dFdC and dFdU were measured at 2h following i.v. (equivalent dose to 15 mg/kg of gemcitabine) or PO administration (80 mg/kg) of Compound **4**, in comparison with i.v. administration of Gemcitabine (15 mg/kg). Strikingly, as can be seen from Fig. 2, there were no detectable dFdC levels in the liver whereas significant levels of dFdU were detected in the liver following i.v. administration of Gemcitabine. In contrast, i.v. and PO administration of Compound **4** resulted in significant levels of dFdC in the liver. Thus, over 1331-fold higher concentration of dFdC was generated following i.v. administration of Compound **4** compared to the same dose Gemcitabine i.v. administration group. Similarly, significantly higher concentration of dFdC was also generated from PO dosing of Compound **4.** Conversely, dFdU levels generated from Compound **4** administration (either i.v. or PO) were much less than that in Gemcitabine i.v. administration group. The overall safety improvement (therapeutic index improvement) of Compound **4** over Gemcitabine was greater than 3500-fold and 2035-fold, for iv and PO dosing of compound **4** respectively (Table 2, showing improvement in Therapeutic Index by Compound **4** over Gemcitabine).

Table 2. Improvement of therapeutic index (II) of prodrugs over Gemcitabine in the liver

| Compounds | [dFdC], fold higher than gemcitabine iv | [dFdU], fold less than gemcitabine iv | TI improvement |
|---|---|---|---|
| Compound 4 IV | >1331 | 2.63 | >3500 |
| Compound 4 PO | >534 | 3.81 | >2035 |

$$TI = [dFdC]\text{-fold improvement} \times [dFdU]\text{-fold improvement}$$

**Pharmacology study**

Human clinical studies of gemcitabine showed potential as part of combination therapies for the treatment of hepatocellular carcinoma, bile duct cancers and pancreatic cancers. In a phase II study, gemcitabine combined with

oxaliplatin and erlotinib as a first line therapy to treat hepatocellular carcinoma showed progression free survivals in 41% of patients at 26 weeks (Cancer Medicine, 2017, 6, 2042-2051). In another phase II study of Chinese patients with metastatic adenocarcinoma of the pancreas, gemcitabine plus nab-paclitaxel treatment showed overall response rate of 35%, which met the preset primary end-point.

**14-day mouse safety study**

A 14-day mouse safety study is conducted to evaluate if a compound of formula I (e.g. Compound **4**) has adverse effects in mice following 14 days of oral gavage dosing and to determine the repeated PK profiles of the prodrug (e.g. Compound **4**) and its metabolites dFdC as well as dFdU.

Male (M) and female (F) CD-1 mice (18-22g of body weight) provided by Beijing Vital River Laboratory Animal Technology Co., Ltd., are randomized into four groups, 5 animals each gender. Animals are dosed with compound **4** by oral gavage. A range of doses is selected in order to determine maximum tolerance dose and dose liming toxicity. A dosing volume of 5 mL/kg is used and administered daily.

| Groups | N, Gender | Treatment | Doses (mg/kg) | Dosing Volume (mL/kg) |
|---|---|---|---|---|
| 1 | 5F+5M | Vehicle | - | 5 |
| 2 | 5F+5M | Compound **4**, low dose | 0.1 | 5 |
| 3 | 5F+5M | Compound **4**, med dose | 1 | 5 |
| 4 | 5F+5M | Compound **4**, high dose | 10 | 5 |

Endpoints including clinical signs, body weight, food consumption, hematology, clinical chemistry, plasma concentrations of Compound **4** and its metabolites (dFdC, dFdU), gross pathology, organ weights are evaluated.

## Claims

**1.** A compound having formula I:

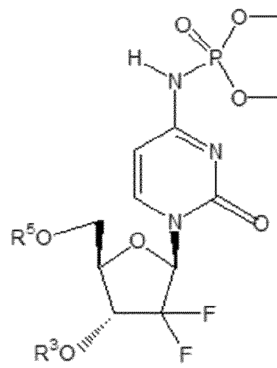

(I),

its stereoisomer, salt, hydrate, solvate, or crystalline form thereof;
wherein,
Ar is optionally substituted phenyl, or optionally substituted 3-pyridyl or 4-pyridyl, $R^3$ is hydrogen or isobutyryl, and $R^5$ is hydrogen or isobutyryl, or $R^3$ and $R^5$ together form a cyclic group as shown below:

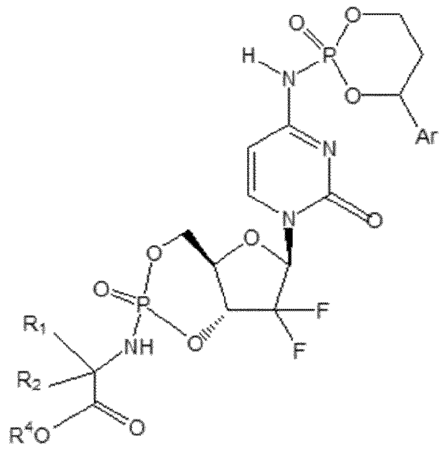

wherein

$R^1$ and $R^2$ are independently selected from the group consisting of H, alkyl and alkylaryl, or $R^1$ and $R^2$ together form an alkylene chain so as to provide, together with the C atom to which they are attached, a cyclic system; and
$R^4$ is selected from the group consisting of alkyl, aryl and alkylaryl.

**2.** The compound according to claim 1 wherein Ar is 3-chlorophenyl, 3-pyridyl, or 4-pyridyl.

**3.** The compound according to claim 1 selected from the group consisting of:

1-((2R,4R,5R)-3,3-difluoro-4-hydroxy-5-(hydroxymethyl)tetrahydrofuran-2-yl)-4-((2-oxido-4-(pyridin-4-yl)-1,3,2-dioxaphosphinan-2-yl)amino)pyrimidin-2(1H)-one;
1-((2R,4R,5R)-3,3-difluoro-4-hydroxy-5-(hydroxymethyl)tetrahydrofuran-2-yl)-4-((2-oxido-4-(pyridin-3-yl)-1,3,2-dioxaphosphinan-2-yl)amino)pyrimidin-2(1H)-one;
4-(((2R,4S)-4-(3-chlorophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;
4-(((2S,4S)-4-(3-chlorophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;
4-((4-(3-bromophenyl)-2-oxido-1,3,2-dioxaphosphinan-2-yl)amino)-1-((2R,4R, SR)-3,3-difluoro-4-hydroxy-5-(hydroxymethyl)tetrahydrofuran-2-yl)pyrimidin-2(1H)-one;
1-((2R,4R,5R)-3,3-difluoro-4-hydroxy-5-(hydroxymethyl)tetrahydrofuran-2-yl)-4-((4-(3-fluorophenyl)-2-oxido-1,3,2-dioxaphosphinan-2-yl)amino)pyrimidin-2(1H)-one;
1- ((2R,4R,5R)-3,3-difluoro-4-hydroxy-5-(hydroxymethyl)tetrahydrofuran-2-yl)-4-((4-(4-methoxyphenyl)-2-oxido-1,3,2-dioxaphosphinan-2-yl)amino)pyrimidin-2(1H)-one; and
((2R,3R,5R)-5-(4-((4-(3-chloro-4-methoxyphenyl)-2-oxido-1,3,2-dioxaphosphinan-2-yl)amino)-2-oxopyrimidin-1(2H)-yl)-4,4-difluoro-3-hydroxytetrahydrofuran-2-yl)methyl pivalate
(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((isobutyryloxy)methyl)tetrahydrofuran-3-yl isobutyrate
((2R,3R,5R)-3-acetoxy-5-(4-((4-(3-chlorophenyl)-2-oxido-1,3,2-dioxaphosphinan-2-yl)amino)-2-oxopyrimidin-1(2H)-yl)-4,4-difluorotetrahydrofuran-2-yl)methyl acetate;
(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((propionyloxy)methyl)tetrahydrofuran-3-yl propionate;
(2R,3R,5R)-5-(4-((4-(3-chlorophenyl)-2-oxido-1,3,2-dioxaphosphinan-2-yl)amino)-2-oxopyrimidin-1(2H)-yl)-4,4-difluoro-2-((2-methoxyacetoxy)methyl)tetrahydrofuran-3-yl 2-methoxyacetate;
(2R,3R, SR)-5-(4-((4-(3-chlorophenyl)-2-oxido-1,3,2-dioxaphosphinan-2-yl)amino)-2-oxopyrimidin-1 (2H)-yl)-4,4-difluoro-2-((pivaloyloxy)methyl)tetrahydrofuran-3-yl pivalate; and
(2R,3R, SR)-5-(4-((4-(3-chlorophenyl)-2-oxido-1,3,2-dioxaphosphinan-2-yl)amino)-2-oxopyrimidin-1(2H)-yl)-4,4-difluoro-2-((isopropyloxycarbonyloxy)methyl)tetrahydrofuran-3-yl isopropyl carbonate.

**4.** A method for preparing a compound of formula I according to any one of claims 1 to 3, comprising the following steps:

(i) protecting 5'-position (5'-OH) of gemcitabine with a suitable protection group to obtain a compound of formula (IV), wherein PG represents a suitable protection group for -OH group;

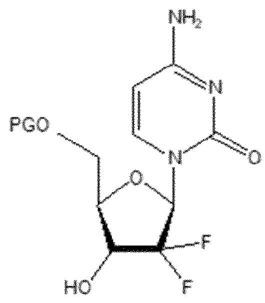

(IV)

(ii) modifying the compound of formula (IV) to obtain a compound of formula (III);

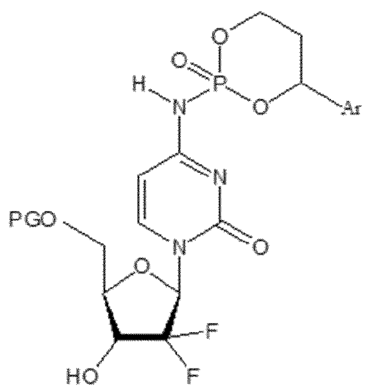

(III)

(iii) removing the protection group (PG) at 5'-position (5'-OH) of gemcitabine to obtain a compound of formula (II);

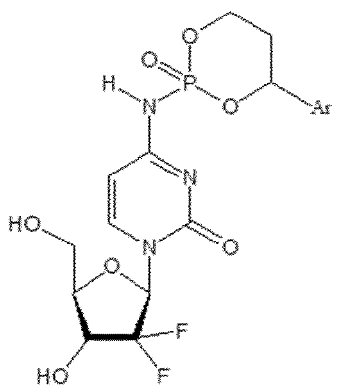

(II)

and, optionally further
(iv) modifying the compound of formula (II) at 3'-position (3'-OH) and/or 5'-position (5'-OH) to obtain the compound of formula (I)

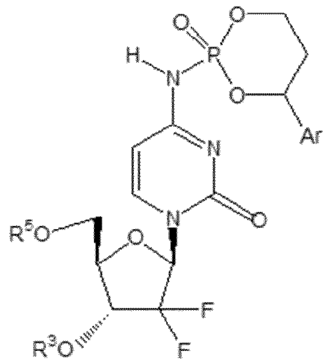

(I),

wherein Ar, $R^3$ and $R^5$ are as defined in any one of claims 1 to 3.

**5.** A compound according to any one of claims 1 to 3 for use in the treatment of susceptible neoplasms, wherein the susceptible neoplasm is selected from the group consisting of the group consisting of T-cell lymphoma, soft tissue sarcoma, pancreatic cancer, breast cancer, Hodgkin's lymphoma, non-Hodgkin's lymphoma, non-small cell lung cancer, ovarian cancer, bladder cancer and Hepatocellular Carcinoma (HCC).

**6.** The compound for use according to claim 5 wherein the treatment comprises administering to a mammal in need thereof a therapeutically effective amount of a compound according to any one of claims 1 to 3 in combination with at least one (preferably one or two) oncolytic agent and/or immune-oncology agent.

**7.** The compound for use according to claim 6, wherein the oncolytic agent is selected from the group consisting of 5-fluorouracil, chloroquine, S-1 (the combination drug tegafur/gimeracil/oteracil), vinorelbine, sorafenib, elpamotide, capecitabine, carboplatin, cisplatin, oxaliplatin, aurora kinase inhibitors (e.g. MSC1992371A), EGFR inhibitors (e.g. erlotinib, gefitinib), tyrosine kinase inhibitors (e.g. lapatinib, vandetanib), topoisomerase inhibitors (e.g. irinotecan, exatecan, Indotecan (aka LMP400) and Indimitecan (aka LMP776), nab-paclitaxel, paclitaxel, docetaxel, pemetrexed, curcumin and radiation therapy.

**8.** The compound for use according to claim 6, wherein the immune-oncology agent is selected from the group consisting of checkpoint inhibitors, PD-1, PD-L1, CTLA-4 and VEGF-A antibodies.

**9.** A pharmaceutical composition comprising a compound according to any one of claims 1 to 3 in combination with a pharmaceutically acceptable carrier, diluent or excipient.

**10.** A method of preparing a pharmaceutical composition comprising the step of combining a compound according to any one of claims 1 to 3 with a pharmaceutically acceptable carrier, diluent or excipient.

**Patentansprüche**

**1.** Verbindung mit der Formel I:

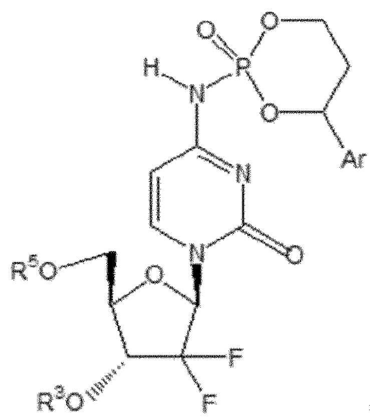

(I),

ihr Stereoisomer, Salz, Hydrat, Solvat oder ihre kristalline Form;
wobei,
Ar ist optional substituiertes Phenyl oder optional substituiertes 3-Pyridyl oder 4-Pyridyl, $R^3$ ist Wasserstoff oder Isobutyryl, und $R^5$ ist Wasserstoff oder Isobutyryl, oder $R^3$ und $R^5$ bilden zusammen eine cyclische Gruppe wie unten gezeigt:

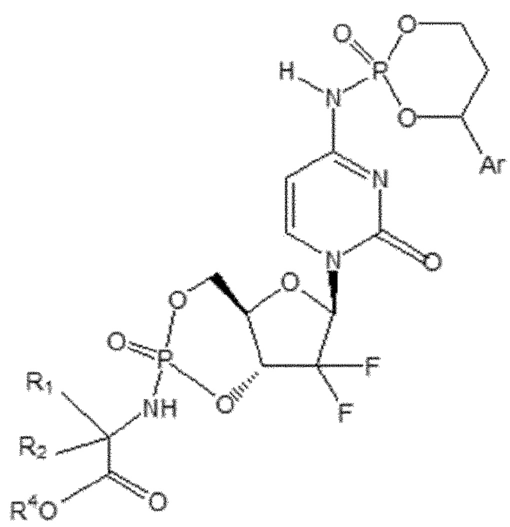

wobei

$R^1$ und $R^2$ unabhängig voneinander ausgewählt sind aus der Gruppe bestehend aus H, Alkyl und Alkylaryl, oder
$R^1$ und $R^2$ zusammen eine Alkylenkette bilden, so dass sie zusammen mit dem C-Atom an das sie gebunden sind, ein cyclisches System bilden; und
$R^4$ ausgewählt ist aus der Gruppe, die aus Alkyl, Aryl und Alkylaryl besteht.

**2.** Verbindung gemäß Anspruch 1, wobei Ar 3-Chlorphenyl, 3-Pyridyl oder 4-Pyridyl ist.

**3.** Verbindung gemäß Anspruch 1, ausgewählt aus der Gruppe bestehend aus:

1-((2R,4R,5R)-3,3-Difluor-4-hydroxy-5-(hydroxymethyl)tetrahydrofuran-2-yl)-4-((2-oxido-4-(pyridin-4-yl)-1,3,2-dioxaphosphinan-2-yl)amino)pyrimidin-2(1H)-on;
1-((2R,4R,5R)-3,3-Difluor-4-hydroxy-5-(hydroxymethyl)tetrahydrofuran-2-yl)-4-((2-oxido-4-(pyridin-3-yl)-1,3,2-dioxaphosphinan-2-yl)amino)pyrimidin-2(1H)-on;
4-(((2R,4S)-4-(3-Chlorphenyl)-2-oxido-1,3,2-dioxaphosphinan-2-yl)amino)-1-((2R,4R,5R)-3,3-difluor-4-hydroxy-5-(hydroxymethyl)tetrahydrofuran-2-yl)pyrimidin-2(1H)-on;
4-(((2S,4S)-4-(3-Chlorphenyl)-2-oxido-1,3,2-dioxaphosphinan-2-yl)amino)-1-((2R,4R,5R)-3,3-difluor-4-hydroxy-5-(hydroxymethyl)tetrahydrofuran-2-yl)pyrimidin-2(1H)-on;
4-((4-(3-Bromphenyl)-2-oxido-l,3,2-dioxaphosphinan-2-yl)amino)-l-((2R,4R,5R)-3,3-difluor-4-hydroxy-5-(hydroxymethyl)tetrahydrofuran-2-yl)pyrimidin-2(1H)-on;
l-((2R,4R,5R)-3,3-Difluor-4-hydroxy-5-(hydroxymethyl)tetrahydrofuran-2-yl)-4-((4-(3-fluorphenyl)-2-oxido-1,3,2-dioxaphosphinan-2-yl)amino)pyrimidin-2(1H)-on;
1-((2R,4R,5R)-3,3-Difluor-4-hydroxy-5-(hydroxymethyl)tetrahydrofuran-2-yl)-4-((4-(4-methoxyphenyl)-2-oxido-1,3,2-dioxaphosphinan-2-yl)amino)pyrimidin-2(1H)-on; und
((2R,3R,5R)-5-(4-((4-(3-Chlor-4-methoxyphenyl)-2-oxido-l,3,2-dioxaphosphinan-2-yl)amino)-2-oxopyrimidin-l(2H)-yl)-4,4-difluor-3-hydroxytetrahydrofuran-2-yl)methylpivalat
(2R,3R,5R)-5-(4-((4-(3-Chlorphenyl)-2-oxido-1,3,2-dioxaphosphinan-2-yl)amino)-2-oxopyrimidin-l(2H)-yl)-4,4-difluor-2-((isobutyryloxy)methyl)tetrahydrofuran-3-yl isobutyrat
((2R,3R,5R)-3-Acetoxy-5-(4-((4-(3-chlorphenyl)-2-oxido-l, 3,2-dioxaphosphinan-2-yl)amino)-2-oxopyrimidin-l(2H)-yl)-4,4-difluortetrahydrofuran-2-yl)methylacetat;
(2R,3R,5R)-5-(4-((4-(3-Chlorphenyl)-2-oxido-l,3,2-dioxaphosphinan-2-yl)amino)-2-oxopyrimidin-1(2H)-yl)-4,4-difluor-2-((propionyloxy)methyl)tetrahydrofuran-3-yl propionat;
(2R,3R,5R)-5-(4-((4-(3-Chlorphenyl)-2-oxido-1,3,2-dioxaphosphinan-2-yl)amino)-2-oxopyrimidin-1(2H)-yl)-4,4-difluor-2-((2-methoxyacetoxy)methyl)tetrahydrofuran-3-yl 2-methoxyacetat;
(2R,3R,5R)-5-(4-((4-(3-Chlorphenyl)-2-oxido-1,3,2-dioxaphosphinan-2-yl)amino)-2-oxopyrimidin-1(2H)-yl)-4,4-difluor-2-((pivaloyloxy)methyl)tetrahydrofuran-3-yl pivalat; und
(2R,3R,5R)-5-(4-((4-(3-Chlorphenyl)-2-oxido-1,3,2-dioxaphosphinan-2-yl)amino)-2-oxopyrimidin-l(2H)-yl)-4,4-difluor-2-((isopropyloxycarbonyloxy)methyl)tetrahydrofuran-3-yl isopropyl carbonat.

**4.** Verfahren zur Herstellung einer Verbindung der Formel I gemäß irgend der Ansprüche 1 bis 3, umfassend die folgenden Schritte:

(i) Schützen der 5'-Position (5'-OH) von Gemcitabin mit einer geeigneten Schutzgruppe, um eine Verbindung der Formel (IV) zu erhalten, wobei PG eine geeignete Schutzgruppe für die -OH-Gruppe darstellt;

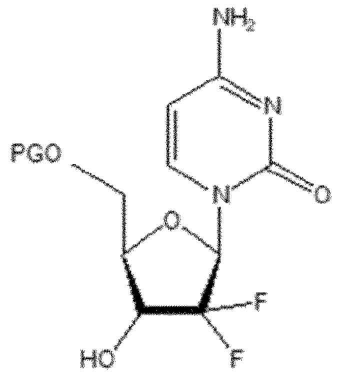

(IV)

(ii) Modifizieren der Verbindung der Formel (IV), um eine Verbindung der Formel (III) zu erhalten;

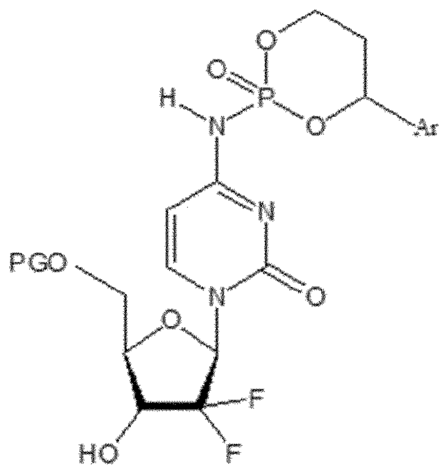

(III)

(iii) Entfernen der Schutzgruppe (PG) an der 5'-Position (5'-OH) von Gemcitabin, um eine Verbindung der Formel (II) zu erhalten;

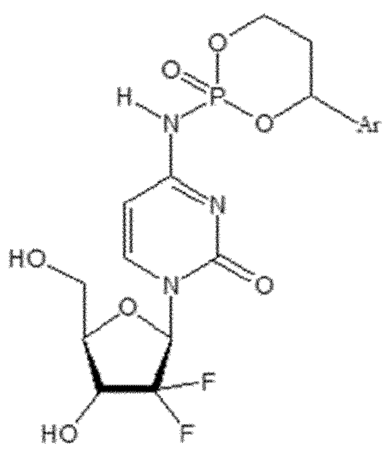

(II)

und, optional weiter

(iv) Modifizieren der Verbindung der Formel (II) in der 3'-Position (3'-OH) und/oder der 5'-Position (5'-OH) um die Verbindung der Formel (I) zu erhalten

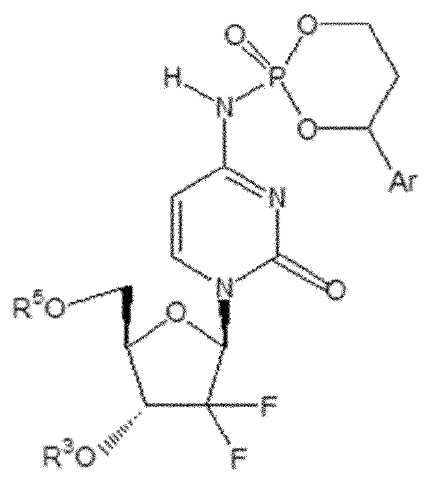

(I),

wobei Ar, $R^3$ und $R^5$ wie in irgendeinem der Ansprüche 1 bis 3 definiert sind.

5. Verbindung gemäß irgendeinem der Ansprüche 1 bis 3 zur Verwendung bei der Behandlung von anfälligen Neoplasmen, wobei das anfällige Neoplasma ausgewählt ist aus der Gruppe bestehend aus T-Zell-Lymphom, Weichteilsarkom, Bauchspeicheldrüsenkrebs, Brustkrebs, Hodgkin-Lymphom, Non-Hodgkin-Lymphom, nicht kleinzelligem Lungenkrebs, Eierstockkrebs, Blasenkrebs und Hepatozelluläres Karzinom (HCC).

6. Verbindung zur Verwendung gemäß Anspruch 5, wobei die Behandlung Verabreichen einer therapeutisch wirksamen Menge einer Verbindung gemäß irgendeinem der Ansprüche 1 bis 3 in Kombination mit mindestens einem (vorzugsweise einem oder zwei) onkolytischen Mittel und/oder einem immunonkologischen Mittel an ein Säugetier, das dies benötigt, umfasst.

7. Verbindung zur Verwendung gemäß Anspruch 6, wobei das onkolytische Mittel ausgewählt ist aus der Gruppe bestehend aus 5-Fluorouracil, Chloroquin, S-1 (dem Kombinationspräparat Tegafur/Gimeracil/Oteracil), Vinorelbin, Sorafenib, Elpamotid, Capecitabin, Carboplatin, Cisplatin, Oxaliplatin, Aurora-Kinase-Inhibitoren (z. B. MSC1992371A), EGFR-Inhibitoren (z. B. Erlotinib, Gefitinib), Tyrosinkinase-Inhibitoren (z. B. Lapatinib, Vandetanib), Topoisomerase-Inhibitoren (z. B. Irinotecan, Exatecan, Indotecan (alias LMP400) und Indimitecan (alias LMP776), nab-Paclitaxel, Paclitaxel, Docetaxel, Pemetrexed, Curcumin und Strahlentherapie.

8. Verbindung zur Verwendung gemäß Anspruch 6, wobei das immunonkologischen Mittel ausgewählt ist aus der Gruppe bestehend aus Checkpoint-Inhibitoren, PD-1, PD-L1, CTLA-4 und VEGF-A-Antikörpern.

9. Pharmazeutische Zusammensetzung, umfassend eine Verbindung gemäß irgendeinem der Ansprüche 1 bis 3 in Kombination mit einem pharmazeutisch akzeptablen Träger, Verdünnungsmittel oder Hilfsstoff.

10. Verfahren zur Herstellung einer pharmazeutischen Zusammensetzung, umfassend den Schritt der Kombination einer Verbindung gemäß irgendeinem der Ansprüche 1 bis 3 mit einem pharmazeutisch verträglichen Träger, Verdünnungsmittel oder Hilfsstoff.

## Revendications

1. Composé ayant la formule I :

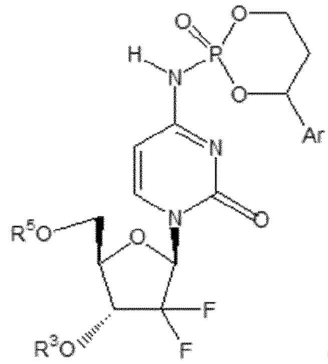

(I),

son stéréoisomère, son sel, son hydrate, son solvate, ou sa forme cristalline ;
dans lequel,
Ar est un phényle facultativement substitué, ou du 3-pyridyle ou 4-pyridyle facultativement substitué, $R^3$ est un hydrogène ou de l'isobutyryle, et $R^5$ est un hydrogène ou de l'isobutyryle, ou $R^3$ et $R^5$ forment ensemble un groupe cyclique comme représenté ci-dessous :

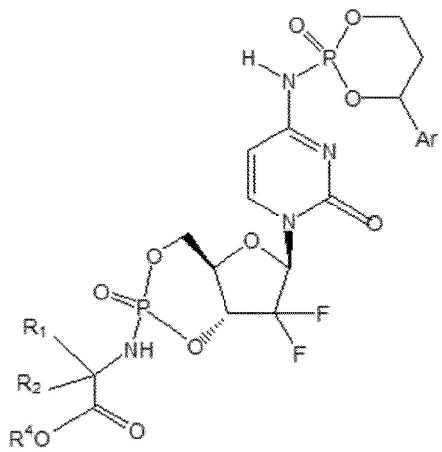

dans lequel

$R^1$ et $R^2$ sont sélectionnés de manière indépendante parmi le groupe consistant en H, alkyle et alkylaryle, ou $R^1$ et $R^2$ forment ensemble une chaîne alkylène de manière à fournir, conjointement avec l'atome C auquel ils sont fixés, un système cyclique ; et
$R^4$ est sélectionné parmi le groupe constitué d'alkyle, aryle et alkylaryle.

**2.** Composé selon la revendication 1 dans lequel Ar est du 3-chlorophényl, du 3-pyridyle, ou du 4-pyridyle.

**3.** Composé selon la revendication 1 sélectionné parmi le groupe consistant en :

1-((2R,4R,5R)-3,3-difluoro-4-hydroxy-5-(hydroxyméthyl)tétrahydrofuran-2-yl)-4-((2-oxido-4-(pyridin-4-yl)-1,3,2-dioxaphosphinan-2-yl)amino)pyrimidin-2(1H)-one ;
1-((2R,4R,5R)-3,3-difluoro-4-hydroxy-5-(hydroxyméthyl)tétrahydrofuran-2-yl)-4-((2-oxido-4-(pyridin-3-yl)-1,3,2-dioxaphosphinan-2-yl)amino)pyrimidin-2(1H)-one ;
4-(((2R,4S)-4-(3-chlorophenyl)-2-oxido-1,3,2-dioxaphosphinan-2-yl)amino)-1-((2R,4R,5R)-3,3-difluoro-4-hydroxy-5-(hydroxyméthyl)tétrahydrofuran-2-yl)pyrimidin-2(1H)-one ;
4-(((2S,4S)-4-(3-chlorophenyl)-2-oxido-1,3,2-dioxaphosphinan-2-yl)amino)-1-((2R,4R,5R)-3,3-difluoro-4-hydroxy-5-(hydroxyméthyl)tétrahydrofuran-2-yl)pyrimidin-2(1H)-one ;
4-((4-(3-bromophenyl)-2-oxido-1,3,2-dioxaphosphinan-2-yl)amino)-1-((2R,4R,5R)-3,3-difluoro-4-hydroxy-5-(hydroxyméthyl)tétrahydrofuran-2-yl)pyrimidin-2(1H)-one ;
1-((2R,4R,5R)-3,3-difluoro-4-hydroxy-5-(hydroxyméthyl)tétrahydrofuran-2-yl)-4-((4-(3-fluorophenyl)-2-oxido-1,3,2-dioxaphosphinan-2-yl)amino)pyrimidin-2(1H)-one ;

1-((2R,4R,5R)-3,3-difluoro-4-hydroxy-5-(hydroxyméthyl)tétrahydrofuran-2-yl)-4-((4-(4-méthoxyphenyl)-2-oxido-1,3,2-dioxaphosphinan-2-yl)amino)pyrimidin-2(1H)-one ;
et
((2R,3R,5R)-5-(4-((4-(3-chloro-4-méthoxyphenyl)-2-oxido-1,3,2-dioxaphosphinan-2-yl)amino)-2-oxopyrimidin-1 (2H)-yl)-4,4-difluoro-3-hydroxytétrahydrofuran-2-yl)méthyl pivalate
(2R,3R,5R)-5-(4-((4-(3-chlorophenyl)-2-oxido-1,3,2-dioxaphosphinan-2-yl)amino)-2-oxopyrimidin-1(2H)-yl)-4,4-difluoro-2-((isobutyryleoxy)méthyl)tétrahydrofuran-3-yl isobutyrate
((2R,3R,5R)-3-acetoxy-5-(4-((4-(3-chlorophenyl)-2-oxido-1,3,2-dioxaphosphinan-2-yl)amino)-2-oxopyrimidin-1(2H)-yl)-4,4-difluorotétrahydrofuran-2-yl)méthyl acétate ;
(2R,3R,5R)-5-(4-((4-(3-chlorophenyl)-2-oxido-1,3,2-dioxaphosphinan-2-yl)amino)-2-oxopyrimidin-1 (2H)-yl)-4,4-difluoro-2-((propionyloxy)méthyl)tétrahydrofuran-3-yl propionate ;
(2R,3R,5R)-5-(4-((4-(3-chlorophenyl)-2-oxido-1,3,2-dioxaphosphinan-2-yl)amino)-2-oxopyrimidin-1 (2H)-yl)-4,4-difluoro-2-((2-méthoxyacetoxy)méthyl)tétrahydrofuran-3-yl 2-méthoxyacétate ;
(2R,3R,5R)-5-(4-((4-(3-chlorophenyl)-2-oxido-1,3,2-dioxaphosphinan-2-yl)amino)-2-oxopyrimidin-1 (2H)-yl)-4,4-difluoro-2-((pivaloyloxy)méthyl)tétrahydrofuran-3-yl pivalate ; et
(2R,3R,5R)-5-(4-((4-(3-chlorophenyl)-2-oxido-1,3,2-dioxaphosphinan-2-yl)amino)-2-oxopyrimidin-1 (2H)-yl)-4,4-difluoro-2-((isopropyloxycarbonyloxy)méthyl)tétrahydrofuran-3-yl isopropyl carbonate.

**4.** Procédé destiné à préparer un composé de formule I selon l'une quelconque des revendications 1 à 3, comprenant les étapes suivantes :

(i) protéger la position 5' (5'-OH) de la gemcitabine avec un groupe de protection approprié pour obtenir un composé de formule (IV), dans lequel PG représente un groupe de protection approprié pour le groupe -OH ;

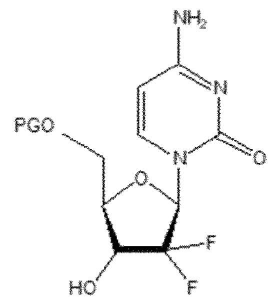

(IV)

(ii)modifier le composé de formule (IV) pour obtenir un composé de formule (III) ;

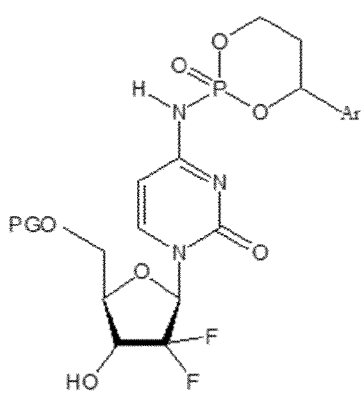

(III)

(iii) supprimer le groupe de protection (PG) en position 5' (5'-OH) de la gemcitabine pour obtenir un composé de formule (II) ;

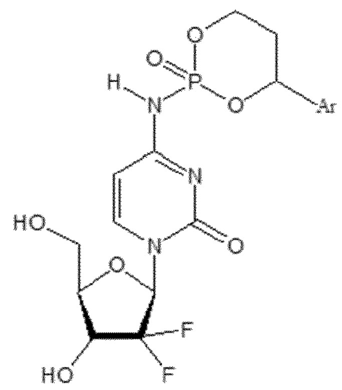

(II)

et facultativement en outre

(iv) modifier le composé de formule (II) en position 3' (3'-OH) et/ou en position 5' (5' - OH) pour obtenir le composé de formule (I)

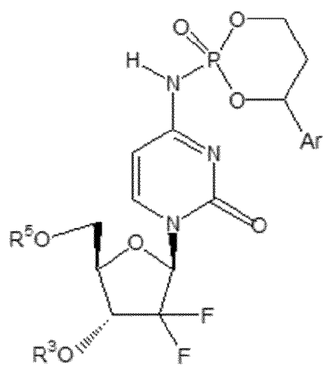

(I),

dans lequel Ar, $R^3$ et $R^5$ sont tels que définis selon l'une quelconque des revendications 1 à 3.

**5.** Composé selon l'une quelconque des revendications 1 à 3, destiné à une utilisation dans le traitement de néoplasmes sensibles, dans lequel le néoplasme sensible est sélectionné parmi le groupe constitué du groupe consistant en un lymphome à cellules T, un sarcome des tissus mous, un cancer du pancréas, un cancer du sein, un lymphome de Hodgkin, un lymphome non hodgkinien, un cancer du poumon à non petites cellules, un cancer de l'ovaire, un cancer de la vessie et un carcinome hépatocellulaire (HCC).

**6.** Composé destiné à une utilisation selon la revendication 5, dans lequel le traitement comprend l'administration à un mammifère qui en a besoin d'une quantité thérapeutiquement efficace d'un composé selon l'une quelconque des revendications 1 à 3 en combinaison avec au moins un (de préférence un ou deux) agent oncolytique et/ou agent immuno-oncologique.

**7.** Composé destiné à une utilisation selon la revendication 6, dans lequel l'agent oncolytique est sélectionné parmi le groupe consistant en 5-fluorouracile, chloroquine, S-1 (la combinaison médicamenteuse tégafur/giméracil/otéracil), vinorelbine, sorafénib, elpamotide, capecitabine, carboplatine, cisplatine, oxaliplatine, inhibiteurs de kinase Aurora (p.ex. MSC1992371A), inhibiteurs EGFR (p.ex. erlotinib, géfitinib), inhibiteurs de tyrosine kinase (p.ex. lapatinib, vandétanib), inhibiteurs de topoisomérase (p.ex. irinotécan, exatécan, Indotécan (aka LMP400) et Indimitécan (aka LMP776), nab-paclitaxel, paclitaxel, docétaxel, pémétrexed, curcumine et radiothérapie.

**8.** Composé destiné à une utilisation selon la revendication 6, dans lequel l'agent immuno-oncologique est sélectionné parmi le groupe constitué d'inhibiteurs de point de contrôle, d'anticorps PD-1, PD-L1, CTLA-4 et VEGF-A.

**9.** Composition pharmaceutique comprenant un composé selon l'une quelconque des revendications 1 à 3 en combinaison avec un véhicule, diluant ou excipient pharmaceutiquement acceptable.

**10.** Procédé destiné à préparer une composition pharmaceutique comprenant l'étape consistant à combiner un composé selon l'une quelconque des revendications 1 à 3 avec un véhicule, diluant ou excipient pharmaceutiquement acceptable.

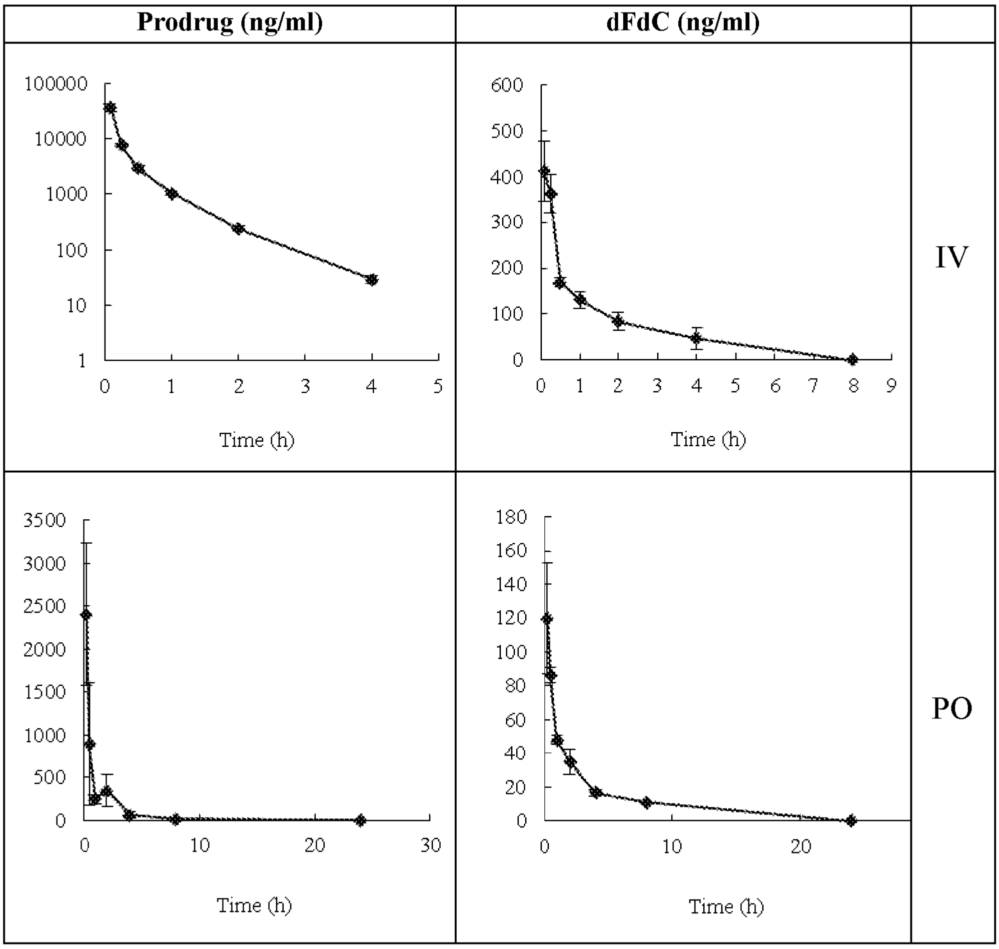
Prodrug (ng/ml)
dFdC (ng/ml)
IV
PO
100000
10000
1000
100
10
1
0 1 2 3 4 5
Time (h)
600
500
400
300
200
100
0
0 1 2 3 4 5 6 7 8 9
Time (h)
3500
3000
2500
2000
1500
1000
500
0
0 10 20 30
Time (h)
180
160
140
120
100
80
60
40
20
0
0 10 20
Time (h)

**FIG. 1**

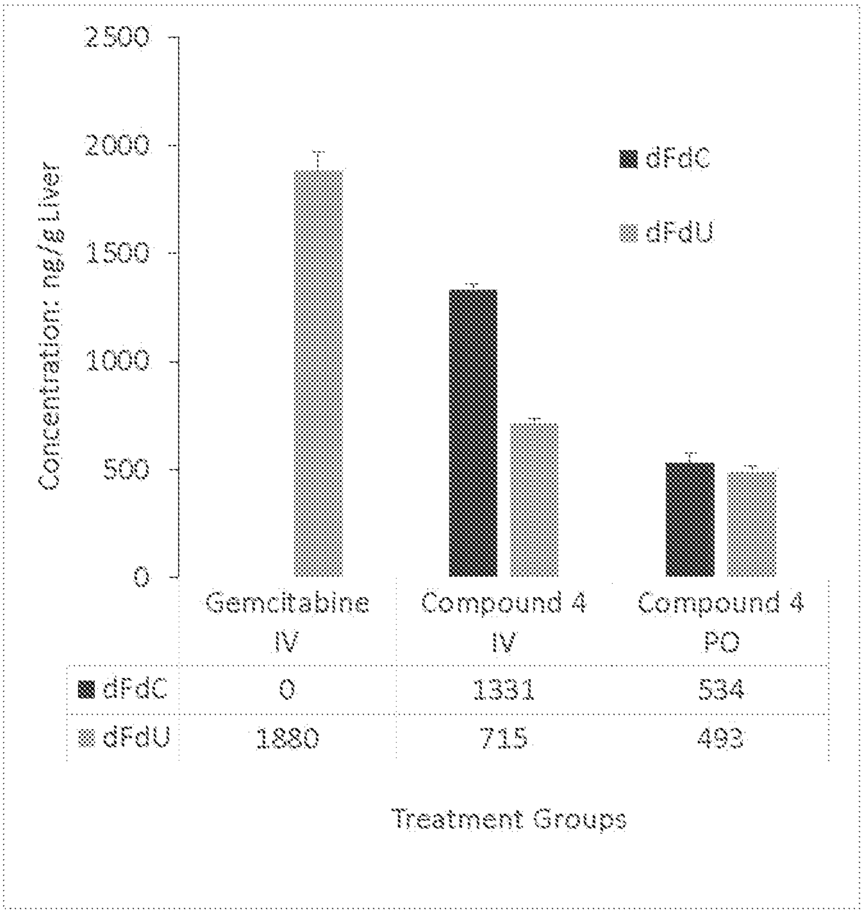
2500
2000
1500
1000
500
0
Concentration: ng/g Liver
dFdC
dFdU
Gemcitabine IV
Compound 4 IV
Compound 4 PO
dFdC 0 1331 534
dFdU 1880 715 493
Treatment Groups

**FIG. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6555518 B
- WO 040412303 A
- WO 9832762 A
- WO 0209768 A
- WO 0276476 A
- WO 0265988 A, Greenwald, Richard m
- US 5420266 A, Britton
- US 5464826 A, Grindey,
- WO 04041203 A, Gallop


### Non-patent literature cited in the description


- *Antimicrobial Agents and Chemotherapy,* 2012, vol. 56, 1942-1948
- *ACS Infect. Dis,* 2017, vol. 3 (1), 45-53
- *Antiviral Research,* 2017, vol. 145, 6-13
- *J. Biol. Chem.,* 2012, vol. 287 (42), 35324-35332
- **SHIPLEY LA.** Metabolism and disposition of gemcitabine, and oncolytic deoxycytidine analog, in mice, mice, and dogs. *Drug Metabolism & Disposition,* 1992, vol. 20 (6), 849-55
- **HORTON ND.** Toxicity of single-dose oral gemcitabine in mice. *American Association for Cancer Research, Poster Presentation,* 27 March 2004
- *J. Med. Chem.,* 2009, vol. 52, 6958-6961
- *Invest. New Drugs,* 2015, vol. 33, 1206-1216
- *Am. J. Clin. Oncol.,* 2012, vol. 35 (5), 418-423
- *Cancer Research on Prevention and Treatment,* 2012, vol. 39 (11), 1369-1372
- *Journal of Hainan Medical University,* 2016, vol. 22 (17), 2029-2031
- *Chinese Clinical Oncology,* 2016, vol. 21 (7), 642-645
- **LIU TW et al.** *Lancet Oncol.,* 2016, vol. 17, 12-4
- *Investigational New Drugs,* 2014, vol. 32 (1), 94-103
- Protecting Groups in Organic Synthesis. Wiley, 1999
- *J. Am. Chem. Soc.,* 2004, vol. 126, 5154-5163
- **THEODORA GREENE ; PETER WUTS.** Protecting Groups in Organic Synthesis. Wiley-Interscience, 1999
- *Journal of Medicinal Chemistry,* 2018, vol. 61, 4904
- *Cancer Medicine,* 2017, vol. 6, 2042-2051